# EUROPEAN PATENT APPLICATION

(11) **EP 4 079 328 A1**
(43) Date of publication of application: **26.10.2022**
(21) Application number: 22163069.2
(22) Date of filing: 05.05.2016
(51) Int. Cl.: A61K 47/68, C07K 14/47, A61K 8/14, C07K 14/54, C07K 16/18, A61K 47/42, A61K 39/395, A61P 35/00, C07K 14/525

(54) **CANCER IMMUNOTHERAPEUTIC**

(30) Priority: 05.05.2015 US 201562157395 P
(62) Divisional of application: 16790111.5
(71) Applicant: Rubicon Biotechnology LLC, Lake Forest, CA 92630 (US)
(72) Inventor: PARSEGHIAN, Missag, Lake Forest, 92630 (US); RICHIERI, Richard, Lake Forest, 92630 (US); REYNOLDS, Glenn, Lake Fores, 92630 (US)
(74) Representative: HGF

(57) **Abstract**

The invention relates to immunotherapeutic compositions and methods for treating specifically solid tumours and cells of the tumour microenvironment.

SEQUENCE LISTING

<110>  Rubicon Biotechnology LLC
       Parseghian, Missag
       Richieri, Richard
       Reynolds, Glenn

<120>  Cancer Immunotherapeutic

<130>  48516-501001WO

<160>  17

<170>  PatentIn version 3.5

<210>  1
<211>  319
<212>  PRT
<213>  Homo sapiens

<400>  1

Ala Gln Val Leu Arg Gly Thr Val Thr Asp Phe Pro Gly Phe Asp Glu
1               5                   10                  15

Arg Ala Asp Ala Glu Thr Leu Arg Lys Ala Met Lys Gly Leu Gly Thr
            20                  25                  30

Asp Glu Glu Ser Ile Leu Thr Leu Leu Thr Ser Arg Ser Asn Ala Gln
        35                  40                  45

Arg Gln Glu Ile Ser Ala Ala Phe Lys Thr Leu Phe Gly Arg Asp Leu
    50                  55                  60

Leu Asp Asp Leu Lys Ser Glu Leu Thr Gly Lys Phe Glu Lys Leu Ile
65                  70                  75                  80

Val Ala Leu Met Lys Pro Ser Arg Leu Tyr Asp Ala Tyr Glu Leu Lys
                85                  90                  95

His Ala Leu Lys Gly Ala Gly Thr Asn Glu Lys Val Leu Thr Glu Ile
            100                 105                 110

Ile Ala Ser Arg Thr Pro Glu Glu Leu Arg Ala Ile Lys Gln Val Tyr
        115                 120                 125

Glu Glu Glu Tyr Gly Ser Ser Leu Glu Asp Asp Val Val Gly Asp Thr
    130                 135                 140

Ser Gly Tyr Tyr Gln Arg Met Leu Val Val Leu Leu Gln Ala Asn Arg
145                 150                 155                 160

Asp Pro Asp Ala Gly Ile Asp Glu Ala Gln Val Glu Gln Asp Ala Gln

                165                 170                 175

Ala Leu Phe Gln Ala Gly Glu Leu Lys Trp Gly Thr Asp Glu Glu Lys
            180                 185                 190

Phe Ile Thr Ile Phe Gly Thr Arg Ser Val Ser His Leu Arg Lys Val
        195                 200                 205

Phe Asp Lys Tyr Met Thr Ile Ser Gly Phe Gln Ile Glu Glu Thr Ile
    210                 215                 220

Asp Arg Glu Thr Ser Gly Asn Leu Glu Gln Leu Leu Leu Ala Val Val
225                 230                 235                 240

Lys Ser Ile Arg Ser Ile Pro Ala Tyr Leu Ala Glu Thr Leu Tyr Tyr
                245                 250                 255

Ala Met Lys Gly Ala Gly Thr Asp Asp His Thr Leu Ile Arg Val Met
            260                 265                 270

Val Ser Arg Ser Glu Ile Asp Leu Phe Asn Ile Arg Lys Glu Phe Arg
        275                 280                 285

Lys Asn Phe Ala Thr Ser Leu Tyr Ser Met Ile Lys Gly Asp Thr Ser
    290                 295                 300

Gly Asp Tyr Lys Lys Ala Leu Leu Leu Leu Cys Gly Glu Asp Asp
305                 310                 315

<210> 2
<211> 318
<212> PRT
<213> Homo sapiens

<400> 2

Lys Gly Gly Pro Gly Ser Ala Val Ser Pro Tyr Pro Thr Phe Asn Pro
1               5                   10                  15

Ser Ser Asp Val Ala Ala Leu His Lys Ala Ile Met Val Lys Gly Val
            20                  25                  30

Asp Glu Ala Thr Ile Ile Asp Ile Leu Thr Lys Arg Asn Asn Ala Gln
        35                  40                  45

Arg Gln Gln Ile Lys Ala Ala Tyr Leu Gln Glu Thr Gly Lys Pro Leu
    50                  55                  60

Asp Glu Thr Leu Lys Lys Ala Leu Thr Gly His Leu Glu Glu Val Val

65                  70                  75                  80

Leu Ala Leu Leu Lys Thr Pro Ala Gln Phe Asp Ala Asp Glu Leu Arg
                85                  90                  95

Ala Ala Met Lys Gly Leu Gly Thr Asp Glu Asp Thr Leu Ile Glu Ile
            100                 105                 110

Leu Ala Ser Arg Thr Asn Lys Glu Ile Arg Asp Ile Asn Arg Val Tyr
        115                 120                 125

Arg Glu Glu Leu Lys Arg Asp Leu Ala Lys Asp Ile Thr Ser Asp Thr
    130                 135                 140

Ser Gly Asp Phe Arg Asn Ala Leu Leu Ser Leu Ala Lys Gly Asp Arg
145                 150                 155                 160

Ser Glu Asp Phe Gly Val Asn Glu Asp Leu Ala Asp Ser Asp Ala Arg
                165                 170                 175

Ala Leu Tyr Glu Ala Gly Glu Arg Arg Lys Gly Thr Asp Val Asn Val
            180                 185                 190

Phe Asn Thr Ile Leu Thr Thr Arg Ser Tyr Pro Gln Leu Arg Arg Val
        195                 200                 205

Phe Gln Lys Tyr Thr Lys Tyr Ser Lys His Asp Met Asn Lys Val Leu
    210                 215                 220

Asp Leu Glu Leu Lys Gly Asp Ile Glu Lys Cys Leu Thr Ala Ile Val
225                 230                 235                 240

Lys Cys Ala Thr Ser Lys Pro Ala Phe Phe Ala Glu Lys Leu His Gln
                245                 250                 255

Ala Met Lys Gly Val Gly Thr Arg His Lys Ala Leu Ile Arg Ile Met
            260                 265                 270

Val Ser Arg Ser Glu Ile Asp Met Asn Asp Ile Lys Ala Phe Tyr Gln
        275                 280                 285

Lys Met Tyr Gly Ile Ser Leu Cys Gln Ala Ile Leu Asp Glu Thr Lys
    290                 295                 300

Gly Asp Tyr Glu Lys Ile Leu Val Ala Leu Cys Gly Gly Asn
305                 310                 315

<210> 3
<211> 320
<212> PRT
<213> Homo sapiens

<400> 3

Pro Pro Ser Ala Tyr Gly Ser Val Lys Ala Tyr Thr Asn Phe Asp Ala
1               5                   10                  15

Glu Arg Asp Ala Leu Asn Ile Glu Thr Ala Ile Lys Thr Lys Gly Val
            20                  25                  30

Asp Glu Val Thr Ile Val Asn Ile Leu Thr Asn Arg Ser Asn Ala Gln
        35                  40                  45

Arg Gln Asp Ile Ala Phe Ala Tyr Gln Arg Arg Thr Lys Lys Glu Leu
    50                  55                  60

Ala Ser Ala Leu Lys Ser Ala Leu Ser Gly His Leu Glu Thr Val Ile
65                  70                  75                  80

Leu Gly Leu Leu Lys Thr Pro Ala Gln Tyr Asp Ala Ser Glu Leu Lys
                85                  90                  95

Ala Ser Met Lys Gly Leu Gly Thr Asp Glu Asp Ser Leu Ile Glu Ile
            100                 105                 110

Ile Cys Ser Arg Thr Asn Gln Glu Leu Gln Glu Ile Asn Arg Val Tyr
        115                 120                 125

Lys Glu Met Tyr Lys Thr Asp Leu Glu Lys Asp Ile Ile Ser Asp Thr
    130                 135                 140

Ser Gly Asp Phe Arg Lys Leu Met Val Ala Leu Ala Lys Gly Arg Arg
145                 150                 155                 160

Ala Glu Asp Gly Ser Val Ile Asp Tyr Glu Leu Ile Asp Gln Asp Ala
                165                 170                 175

Arg Asp Leu Tyr Asp Ala Gly Val Lys Arg Lys Gly Thr Asp Val Pro
            180                 185                 190

Lys Trp Ile Ser Ile Met Thr Glu Arg Ser Val Pro His Leu Gln Lys
        195                 200                 205

Val Phe Asp Arg Tyr Lys Ser Tyr Ser Pro Tyr Asp Met Leu Glu Ser
    210                 215                 220

Ile Arg Lys Glu Val Lys Gly Asp Leu Glu Asn Ala Phe Leu Asn Leu
225                 230                 235                 240

Val Gln Cys Ile Gln Asn Lys Pro Leu Tyr Phe Ala Asp Arg Leu Tyr
                245                 250                 255

Asp Ser Met Lys Gly Lys Gly Thr Arg Asp Lys Val Leu Ile Arg Ile
            260                 265                 270

Met Val Ser Arg Ser Glu Val Asp Met Leu Lys Ile Arg Ser Glu Phe
        275                 280                 285

Lys Arg Lys Tyr Gly Lys Ser Leu Tyr Tyr Tyr Ile Gln Gln Asp Thr
    290                 295                 300

Lys Gly Asp Tyr Gln Lys Ala Leu Leu Tyr Leu Cys Gly Gly Asp Asp
305                 310                 315                 320

<210>  4
<211>  319
<212>  PRT
<213>  Homo sapiens

<400>  4

Trp Val Gly His Arg Gly Thr Val Arg Asp Tyr Pro Asp Phe Ser Pro
1               5                   10                  15

Ser Val Asp Ala Glu Ala Ile Gln Lys Ala Ile Arg Gly Ile Gly Thr
            20                  25                  30

Asp Glu Lys Met Leu Ile Ser Ile Leu Thr Glu Arg Ser Asn Ala Gln
        35                  40                  45

Arg Gln Leu Ile Val Lys Glu Tyr Gln Ala Ala Tyr Gly Lys Glu Leu
    50                  55                  60

Lys Asp Asp Leu Lys Gly Asp Leu Ser Gly His Phe Glu His Leu Met
65                  70                  75                  80

Val Ala Leu Val Thr Pro Pro Ala Val Phe Asp Ala Lys Gln Leu Lys
                85                  90                  95

Lys Ser Met Lys Gly Ala Gly Thr Asn Glu Asp Ala Leu Ile Glu Ile
            100                 105                 110

Leu Thr Thr Arg Thr Ser Arg Gln Met Lys Asp Ile Ser Gln Ala Tyr
        115                 120                 125

Tyr Thr Val Tyr Lys Lys Ser Leu Gly Asp Asp Ile Ser Ser Glu Thr
    130                 135                 140

Ser Gly Asp Phe Arg Lys Ala Leu Leu Thr Leu Ala Asp Gly Arg Arg
145                 150                 155                 160

Asp Glu Ser Leu Lys Val Asp Glu His Leu Ala Lys Gln Asp Ala Gln
                165                 170                 175

Ile Leu Tyr Lys Ala Gly Glu Asn Arg Trp Gly Thr Asp Glu Asp Lys
            180                 185                 190

Phe Thr Glu Ile Leu Cys Leu Arg Ser Phe Pro Gln Leu Lys Leu Thr
        195                 200                 205

Phe Asp Glu Tyr Arg Asn Ile Ser Gln Lys Asp Ile Val Asp Ser Ile
    210                 215                 220

Lys Gly Glu Leu Ser Gly His Phe Glu Asp Leu Leu Leu Ala Ile Val
225                 230                 235                 240

Asn Cys Val Arg Asn Thr Pro Ala Phe Leu Ala Glu Arg Leu His Arg
                245                 250                 255

Ala Leu Lys Gly Ile Gly Thr Asp Glu Phe Thr Leu Asn Arg Ile Met
            260                 265                 270

Val Ser Arg Ser Glu Ile Asp Leu Leu Asp Ile Arg Thr Glu Phe Lys
        275                 280                 285

Lys His Tyr Gly Tyr Ser Leu Tyr Ser Ala Ile Lys Ser Asp Thr Ser
    290                 295                 300

Gly Asp Tyr Glu Ile Thr Leu Leu Lys Ile Cys Gly Gly Asp Asp
305                 310                 315

<210>  5
<211>  318
<212>  PRT
<213>  Homo sapiens

<400>  5

Ala Thr Lys Gly Gly Thr Val Lys Ala Ala Ser Gly Phe Asn Ala Met
1               5                   10                  15

Glu Asp Ala Gln Thr Leu Arg Lys Ala Met Lys Gly Leu Gly Thr Asp
            20                  25                  30

Glu Asp Ala Ile Ile Ser Val Leu Ala Tyr Arg Asn Thr Ala Gln Arg
        35                  40                  45

Gln Glu Ile Arg Thr Ala Tyr Lys Ser Thr Ile Gly Arg Asp Leu Ile
    50                  55                  60

Asp Asp Leu Lys Ser Glu Leu Ser Gly Asn Phe Glu Gln Val Ile Val
65                  70                  75                  80

Gly Met Met Thr Pro Thr Val Leu Tyr Asp Val Gln Glu Leu Arg Arg
                85                  90                  95

Ala Met Lys Gly Ala Gly Thr Asp Glu Gly Cys Leu Ile Glu Ile Leu
            100                 105                 110

Ala Ser Arg Thr Pro Glu Glu Ile Arg Arg Ile Ser Gln Thr Tyr Gln
        115                 120                 125

Gln Gln Tyr Gly Arg Ser Leu Glu Asp Asp Ile Arg Ser Asp Thr Ser
    130                 135                 140

Phe Met Phe Gln Arg Val Leu Val Ser Leu Ser Ala Gly Gly Arg Asp
145                 150                 155                 160

Glu Gly Asn Tyr Leu Asp Asp Ala Leu Val Arg Gln Asp Ala Gln Asp
                165                 170                 175

Leu Tyr Glu Ala Gly Glu Lys Lys Trp Gly Thr Asp Glu Val Lys Phe
            180                 185                 190

Leu Thr Val Leu Cys Ser Arg Asn Arg Asn His Leu Leu His Val Phe
        195                 200                 205

Asp Glu Tyr Lys Arg Ile Ser Gln Lys Asp Ile Glu Gln Ser Ile Lys
    210                 215                 220

Ser Glu Thr Ser Gly Ser Phe Glu Asp Ala Leu Leu Ala Ile Val Lys
225                 230                 235                 240

Cys Met Arg Asn Lys Ser Ala Tyr Phe Ala Glu Lys Leu Tyr Lys Ser
                245                 250                 255

Met Lys Gly Leu Gly Thr Asp Asp Asn Thr Leu Ile Arg Val Met Val
            260                 265                 270

Ser Arg Ala Glu Ile Asp Met Leu Asp Ile Arg Ala His Phe Lys Arg
        275                 280                 285

Leu Tyr Gly Lys Ser Leu Tyr Ser Phe Ile Lys Gly Asp Thr Ser Gly
    290                 295                 300

Asp Tyr Arg Lys Val Leu Leu Val Leu Cys Gly Gly Asp Asp
305                 310                 315

<210>  6
<211>  319
<212>  PRT
<213>  Homo sapiens

<400>  6

Ala Gln Val Leu Arg Gly Thr Val Thr Asp Phe Pro Gly Phe Asp Glu
1               5                   10                  15

Arg Ala Asp Ala Glu Thr Leu Arg Lys Ala Met Lys Gly Leu Gly Thr
            20                  25                  30

Asp Glu Glu Ser Ile Leu Thr Leu Leu Thr Ser Arg Ser Asn Ala Gln
        35                  40                  45

Arg Gln Glu Ile Ser Ala Ala Phe Lys Thr Leu Phe Gly Arg Asp Leu
    50                  55                  60

Leu Asp Asp Leu Lys Ser Glu Leu Thr Gly Lys Phe Glu Lys Leu Ile
65                  70                  75                  80

Val Ala Leu Met Lys Pro Ser Arg Leu Tyr Asp Ala Tyr Glu Leu Lys
                85                  90                  95

His Ala Leu Lys Gly Ala Gly Thr Asn Glu Lys Val Leu Thr Glu Ile
            100                 105                 110

Ile Ala Ser Arg Thr Pro Glu Glu Leu Arg Ala Ile Lys Gln Val Tyr
        115                 120                 125

Glu Glu Glu Tyr Gly Ser Ser Leu Glu Asp Asp Val Val Gly Asp Thr
    130                 135                 140

Ser Gly Tyr Tyr Gln Arg Met Leu Val Val Leu Leu Gln Ala Asn Arg
145                 150                 155                 160

Asp Pro Asp Ala Gly Ile Asp Glu Ala Gln Val Glu Gln Asp Ala Gln
                165                 170                 175

Ala Leu Phe Gln Ala Gly Glu Leu Lys Trp Gly Thr Asp Glu Glu Lys
            180                 185                 190

Phe Ile Thr Ile Phe Gly Thr Arg Ser Val Ser His Leu Arg Lys Val
        195                 200                 205

Phe Asp Lys Tyr Met Thr Ile Ser Gly Phe Gln Ile Glu Glu Thr Ile
    210                 215                 220

Asp Arg Glu Thr Ser Gly Asn Leu Glu Gln Leu Leu Leu Ala Val Val
225                 230                 235                 240

Lys Ser Ile Arg Ser Ile Pro Ala Tyr Leu Ala Glu Thr Leu Tyr Tyr
                245                 250                 255

Ala Met Lys Gly Ala Gly Thr Asp Asp His Thr Leu Ile Arg Val Met
            260                 265                 270

Val Ser Arg Ser Glu Ile Asp Leu Phe Asn Ile Arg Lys Glu Phe Arg
        275                 280                 285

Lys Asn Phe Ala Thr Ser Leu Tyr Ser Met Ile Lys Gly Asp Thr Ser
    290                 295                 300

Gly Asp Tyr Lys Lys Ala Leu Leu Leu Leu Cys Gly Glu Asp Asp
305                 310                 315

<210>  7
<211>  324
<212>  PRT
<213>  Homo sapiens

<400>  7

Arg Val Glu Leu Lys Gly Thr Val Arg Pro Ala Asn Asp Phe Asn Pro
1               5                   10                  15

Asp Ala Asp Ala Lys Ala Leu Arg Lys Ala Met Lys Gly Leu Gly Thr
            20                  25                  30

Asp Glu Asp Thr Ile Ile Asp Ile Ile Thr His Arg Ser Asn Val Gln
        35                  40                  45

Arg Gln Gln Ile Arg Gln Thr Phe Lys Ser His Phe Gly Arg Asp Leu
    50                  55                  60

Met Thr Asp Leu Lys Ser Glu Ile Ser Gly Asp Leu Ala Arg Leu Ile
65                  70                  75                  80

Leu Gly Leu Met Met Pro Pro Ala His Tyr Asp Ala Lys Gln Leu Lys
                85                  90                  95

Lys Ala Met Glu Gly Ala Gly Thr Asp Glu Lys Ala Leu Ile Glu Ile
            100                 105                 110

Leu Ala Thr Arg Thr Asn Ala Glu Ile Arg Ala Ile Asn Glu Ala Tyr
        115                 120                 125

Lys Glu Asp Tyr His Lys Ser Leu Glu Asp Ala Leu Ser Ser Asp Thr
    130                 135                 140

Ser Gly His Phe Arg Arg Ile Leu Ile Ser Leu Ala Thr Gly His Arg
145                 150                 155                 160

Glu Glu Gly Gly Glu Asn Leu Asp Gln Ala Arg Glu Asp Ala Gln Val
                165                 170                 175

Ala Ala Glu Ile Leu Glu Ile Ala Asp Thr Pro Ser Gly Asp Lys Thr
            180                 185                 190

Ser Leu Glu Thr Arg Phe Met Thr Ile Leu Cys Thr Arg Thr Tyr Pro
        195                 200                 205

His Leu Arg Arg Val Phe Gln Glu Phe Ile Lys Met Thr Asn Tyr Asp
    210                 215                 220

Val Glu His Thr Ile Lys Lys Glu Met Ser Gly Asp Val Arg Asp Ala
225                 230                 235                 240

Phe Val Ala Ile Val Gln Ser Val Lys Asn Lys Pro Leu Phe Phe Ala
                245                 250                 255

Asp Lys Leu Tyr Lys Ser Met Lys Gly Ala Gly Thr Asp Glu Lys Thr
            260                 265                 270

Leu Thr Arg Ile Met Val Ser Arg Ser Glu Ile Asp Leu Leu Asn Ile
        275                 280                 285

Arg Arg Glu Phe Ile Glu Lys Tyr Asp Lys Ser Leu His Gln Ala Ile
    290                 295                 300

Glu Gly Asp Thr Ser Gly Asp Phe Leu Lys Ala Leu Leu Ala Leu Cys
305                 310                 315                 320

Gly Gly Glu Asp

<210> 8
<211> 317

<212>  PRT
<213>  Homo sapiens

<400>  8

Thr Gln Val Thr Gln Gly Thr Ile Arg Pro Ala Ala Asn Phe Asp Ala
1               5                   10                  15

Ile Arg Asp Ala Glu Ile Leu Arg Lys Ala Met Lys Gly Phe Gly Thr
            20                  25                  30

Asp Glu Gln Ala Ile Val Asp Val Val Ala Asn Arg Ser Asn Asp Gln
        35                  40                  45

Arg Gln Lys Ile Lys Ala Ala Phe Lys Thr Ser Tyr Gly Lys Asp Leu
    50                  55                  60

Ile Lys Asp Leu Lys Ser Glu Leu Ser Gly Asn Met Glu Glu Leu Ile
65                  70                  75                  80

Leu Ala Leu Phe Met Pro Pro Thr Tyr Tyr Asp Ala Trp Ser Leu Arg
                85                  90                  95

Lys Ala Met Gln Gly Ala Gly Thr Gln Glu Arg Val Leu Ile Glu Ile
            100                 105                 110

Leu Cys Thr Arg Thr Asn Gln Glu Ile Arg Glu Ile Val Arg Cys Tyr
        115                 120                 125

Gln Ser Glu Phe Gly Arg Asp Leu Glu Lys Asp Ile Arg Ser Asp Thr
    130                 135                 140

Ser Gly His Phe Glu Arg Leu Leu Val Ser Met Cys Gln Gly Asn Arg
145                 150                 155                 160

Asp Glu Asn Gln Ser Ile Asn His Gln Met Ala Gln Glu Asp Ala Gln
                165                 170                 175

Arg Leu Tyr Gln Ala Gly Glu Gly Arg Leu Gly Thr Asp Glu Ser Cys
            180                 185                 190

Phe Asn Met Ile Leu Ala Thr Arg Ser Phe Pro Gln Leu Arg Ala Thr
        195                 200                 205

Met Glu Ala Tyr Ser Arg Met Ala Asn Arg Asp Leu Leu Ser Ser Val
    210                 215                 220

Ser Arg Glu Phe Ser Gly Tyr Val Glu Ser Gly Leu Lys Thr Ile Leu
225                 230                 235                 240

Gln Cys Ala Leu Asn Arg Pro Ala Phe Phe Ala Glu Arg Leu Tyr Tyr
                245                 250                 255

Ala Met Lys Gly Ala Gly Thr Asp Asp Ser Thr Leu Val Arg Ile Val
            260                 265                 270

Val Thr Arg Ser Glu Ile Asp Leu Val Gln Ile Lys Gln Met Phe Ala
        275                 280                 285

Gln Met Tyr Gln Lys Thr Leu Gly Thr Met Ile Ala Gly Asp Thr Ser
    290                 295                 300

Gly Asp Tyr Arg Arg Leu Leu Leu Ala Ile Val Gly Gln
305                 310                 315

<210>  9
<211>  320
<212>  PRT
<213>  Homo sapiens

<400>  9

Ile Glu Gln Glu Gly Val Thr Val Lys Ser Ser Ser His Phe Asn Pro
1               5                   10                  15

Asp Pro Asp Ala Glu Thr Leu Tyr Lys Ala Met Lys Gly Ile Gly Thr
            20                  25                  30

Asn Glu Gln Ala Ile Ile Asp Val Leu Thr Lys Arg Ser Asn Thr Gln
        35                  40                  45

Arg Gln Gln Ile Ala Lys Ser Phe Lys Ala Gln Phe Gly Lys Asp Leu
    50                  55                  60

Thr Glu Thr Leu Lys Ser Glu Leu Ser Gly Lys Phe Glu Arg Leu Ile
65                  70                  75                  80

Val Ala Leu Met Tyr Pro Pro Tyr Arg Tyr Glu Ala Lys Glu Leu His
                85                  90                  95

Asp Ala Met Lys Gly Leu Gly Thr Lys Glu Gly Val Ile Ile Glu Ile
            100                 105                 110

Leu Ala Ser Arg Thr Lys Asn Gln Leu Arg Glu Ile Met Lys Ala Tyr
        115                 120                 125

Glu Glu Asp Tyr Gly Ser Ser Leu Glu Glu Asp Ile Gln Ala Asp Thr
    130                 135                 140

Ser Gly Tyr Leu Glu Arg Ile Leu Val Cys Leu Leu Gln Gly Ser Arg
145                 150                 155                 160

Asp Asp Val Ser Ser Phe Val Asp Pro Ala Leu Ala Leu Gln Asp Ala
                165                 170                 175

Gln Asp Leu Tyr Ala Ala Gly Glu Lys Ile Arg Gly Thr Asp Glu Met
            180                 185                 190

Lys Phe Ile Thr Ile Leu Cys Thr Arg Ser Ala Thr His Leu Leu Arg
        195                 200                 205

Val Phe Glu Glu Tyr Glu Lys Ile Ala Asn Lys Ser Ile Glu Asp Ser
    210                 215                 220

Ile Lys Ser Glu Thr His Gly Ser Leu Glu Glu Ala Met Leu Thr Val
225                 230                 235                 240

Val Lys Cys Thr Gln Asn Leu His Ser Tyr Phe Ala Glu Arg Leu Tyr
                245                 250                 255

Tyr Ala Met Lys Gly Ala Gly Thr Arg Asp Gly Thr Leu Ile Arg Asn
            260                 265                 270

Ile Val Ser Arg Ser Glu Ile Asp Leu Asn Leu Ile Lys Cys His Phe
        275                 280                 285

Lys Lys Met Tyr Gly Lys Thr Leu Ser Ser Met Ile Met Glu Asp Thr
    290                 295                 300

Ser Gly Asp Tyr Lys Asn Ala Leu Leu Ser Leu Val Gly Ser Asp Pro
305                 310                 315                 320

<210>  10
<211>  318
<212>  PRT
<213>  Homo sapiens

<400>  10

Ala Trp Gly Thr Leu Gly Thr Leu Arg Thr Phe Leu Asn Phe Ser Val
1               5                   10                  15

Asp Lys Asp Ala Gln Arg Leu Leu Arg Ala Ile Thr Gly Gln Gly Val
            20                  25                  30

Asp Arg Ser Ala Ile Val Asp Val Leu Thr Asn Arg Ser Arg Glu Gln
        35                  40                  45

Arg Gln Leu Ile Ser Arg Asn Phe Gln Glu Arg Thr Gln Gln Asp Leu
    50                  55                  60

Met Lys Ser Leu Gln Ala Ala Leu Ser Gly Asn Leu Glu Arg Ile Val
65                  70                  75                  80

Met Ala Leu Leu Gln Pro Thr Ala Gln Phe Asp Ala Gln Glu Leu Arg
                85                  90                  95

Thr Ala Leu Lys Ala Ser Asp Ser Ala Val Asp Val Ala Ile Glu Ile
            100                 105                 110

Leu Ala Thr Arg Thr Pro Pro Gln Leu Gln Glu Cys Leu Ala Val Tyr
        115                 120                 125

Lys His Asn Phe Gln Val Glu Ala Val Asp Gly Ile Thr Ser Glu Thr
    130                 135                 140

Ser Gly Ile Leu Gln Asp Leu Leu Leu Ala Leu Ala Lys Gly Gly Arg
145                 150                 155                 160

Asp Ser Tyr Ser Gly Ile Ile Asp Tyr Asn Leu Ala Glu Gln Asp Val
                165                 170                 175

Gln Ala Leu Gln Arg Ala Glu Gly Pro Ser Arg Glu Glu Thr Trp Val
            180                 185                 190

Pro Val Phe Thr Gln Arg Asn Pro Glu His Leu Ile Arg Val Phe Asp
        195                 200                 205

Gln Tyr Gln Arg Ser Thr Gly Gln Glu Leu Glu Glu Ala Val Gln Asn
    210                 215                 220

Arg Phe His Gly Asp Ala Gln Val Ala Leu Leu Gly Leu Ala Ser Val
225                 230                 235                 240

Ile Lys Asn Thr Pro Leu Tyr Phe Ala Asp Lys Leu His Gln Ala Leu
                245                 250                 255

Gln Glu Thr Glu Pro Asn Tyr Gln Val Leu Ile Arg Ile Leu Ile Ser
            260                 265                 270

Arg Cys Glu Thr Asp Leu Leu Ser Ile Arg Ala Glu Phe Arg Lys Lys
        275                 280                 285

Phe Gly Lys Ser Leu Tyr Ser Ser Leu Gln Asp Ala Val Lys Gly Asp

290                 295                 300

Cys Gln Ser Ala Leu Leu Ala Leu Cys Arg Ala Glu Asp Met
305                 310                 315

<210>  11
<211>  321
<212>  PRT
<213>  Homo sapiens

<400>  11

Gly Asp Tyr Val Gln Gly Thr Ile Phe Pro Ala Pro Asn Phe Asn Pro
1               5                   10                  15

Ile Met Asp Ala Gln Met Leu Gly Gly Ala Leu Gln Gly Phe Asp Cys
            20                  25                  30

Asp Lys Asp Met Leu Ile Asn Ile Leu Thr Gln Arg Cys Asn Ala Gln
        35                  40                  45

Arg Met Met Ile Ala Glu Ala Tyr Gln Ser Met Tyr Gly Arg Asp Leu
    50                  55                  60

Ile Gly Asp Leu Arg Glu Gln Leu Ser Asp His Phe Lys Asp Val Met
65                  70                  75                  80

Ala Gly Leu Met Tyr Pro Pro Pro Leu Tyr Asp Ala His Glu Leu Trp
                85                  90                  95

His Ala Met Lys Gly Val Gly Thr Asp Glu Asn Cys Leu Ile Glu Ile
            100                 105                 110

Leu Ala Ser Arg Thr Asn Gly Glu Ile Phe Gln Met Arg Glu Ala Tyr
        115                 120                 125

Cys Leu Gln Tyr Ser Asn Asn Leu Gln Glu Asp Ile Tyr Ser Glu Thr
    130                 135                 140

Ser Gly His Phe Arg Asp Thr Leu Met Asn Leu Val Gln Gly Thr Arg
145                 150                 155                 160

Glu Glu Gly Tyr Thr Asp Pro Ala Met Ala Ala Gln Asp Ala Met Val
                165                 170                 175

Leu Trp Glu Ala Cys Gln Gln Lys Thr Gly Glu His Lys Thr Met Leu
            180                 185                 190

Gln Met Ile Leu Cys Asn Lys Ser Tyr Gln Gln Leu Arg Leu Val Phe

195                 200                 205

Gln Glu Phe Gln Asn Ile Ser Gly Gln Asp Met Val Asp Ala Ile Asn
    210                 215                 220

Glu Cys Tyr Asp Gly Tyr Phe Gln Glu Leu Leu Val Ala Ile Val Leu
225                 230                 235                 240

Cys Val Arg Asp Lys Pro Ala Tyr Phe Ala Tyr Arg Leu Tyr Ser Ala
                245                 250                 255

Ile His Asp Phe Gly Phe His Asn Lys Thr Val Ile Arg Ile Leu Ile
            260                 265                 270

Ala Arg Ser Glu Ile Asp Leu Leu Thr Ile Arg Lys Arg Tyr Lys Glu
        275                 280                 285

Arg Tyr Gly Lys Ser Leu Phe His Asp Ile Arg Asn Phe Ala Ser Gly
    290                 295                 300

His Tyr Lys Lys Ala Leu Leu Ala Ile Cys Ala Gly Asp Ala Glu Asp
305                 310                 315                 320

Tyr

<210>  12
<211>  319
<212>  PRT
<213>  Homo sapiens

<400>  12

Gln Phe Gly Ser Arg Gly Thr Ile Thr Asp Ala Pro Gly Phe Asp Pro
1               5                   10                  15

Leu Arg Asp Ala Glu Val Leu Arg Lys Ala Met Lys Gly Phe Gly Thr
            20                  25                  30

Asp Glu Gln Ala Ile Ile Asp Cys Leu Gly Ser Arg Ser Asn Lys Gln
        35                  40                  45

Arg Gln Gln Ile Leu Leu Ser Phe Lys Thr Ala Tyr Gly Lys Asp Leu
    50                  55                  60

Ile Lys Asp Leu Lys Ser Glu Leu Ser Gly Asn Phe Glu Lys Thr Ile
65                  70                  75                  80

Leu Ala Leu Met Lys Thr Pro Val Leu Phe Asp Ile Tyr Glu Ile Lys

                85                  90                  95

Glu Ala Ile Lys Gly Val Gly Thr Asp Glu Ala Cys Leu Ile Glu Ile
            100                 105                 110

Leu Ala Ser Arg Ser Asn Glu His Ile Arg Glu Leu Asn Arg Ala Tyr
        115                 120                 125

Lys Ala Glu Phe Lys Lys Thr Leu Glu Glu Ala Ile Arg Ser Asp Thr
    130                 135                 140

Ser Gly His Phe Gln Arg Leu Leu Ile Ser Leu Ser Gln Gly Asn Arg
145                 150                 155                 160

Asp Glu Ser Thr Asn Val Asp Met Ser Leu Ala Gln Arg Asp Ala Gln
                165                 170                 175

Glu Leu Tyr Ala Ala Gly Glu Asn Arg Leu Gly Thr Asp Glu Ser Lys
            180                 185                 190

Phe Asn Ala Val Leu Cys Ser Arg Ser Arg Ala His Leu Val Ala Val
        195                 200                 205

Phe Asn Glu Tyr Gln Arg Met Thr Gly Arg Asp Ile Glu Lys Ser Ile
    210                 215                 220

Cys Arg Glu Met Ser Gly Asp Leu Glu Glu Gly Met Leu Ala Val Val
225                 230                 235                 240

Lys Cys Leu Lys Asn Thr Pro Ala Phe Phe Ala Glu Arg Leu Asn Lys
                245                 250                 255

Ala Met Arg Gly Ala Gly Thr Lys Asp Arg Thr Leu Ile Arg Ile Met
            260                 265                 270

Val Ser Arg Ser Glu Thr Asp Leu Leu Asp Ile Arg Ser Glu Tyr Lys
        275                 280                 285

Arg Met Tyr Gly Lys Ser Leu Tyr His Asp Ile Ser Gly Asp Thr Ser
    290                 295                 300

Gly Asp Tyr Arg Lys Ile Leu Leu Lys Ile Cys Gly Gly Asn Asp
305                 310                 315

<210> 13
<211> 314
<212> PRT
<213> Homo sapiens

<400>  13

Gly Asn Arg His Ala Lys Ala Ser Ser Pro Gln Gly Phe Asp Val Asp
1               5                   10                  15

Arg Asp Ala Lys Lys Leu Asn Lys Ala Cys Lys Gly Met Gly Thr Asn
            20                  25                  30

Glu Ala Ala Ile Ile Glu Ile Leu Ser Gly Arg Thr Ser Asp Glu Arg
        35                  40                  45

Gln Gln Ile Lys Gln Lys Tyr Lys Ala Thr Tyr Gly Asn Arg His Ala
    50                  55                  60

Lys Ala Ser Ser Pro Gln Gly Phe Asp Val Asp Arg Asp Ala Lys Lys
65                  70                  75                  80

Leu Asn Lys Ala Cys Lys Gly Met Gly Thr Asn Glu Ala Ala Ile Ile
                85                  90                  95

Glu Ile Leu Ser Gly Arg Thr Ser Asp Glu Arg Gln Gln Ile Lys Gln
            100                 105                 110

Lys Tyr Lys Ala Thr Tyr Ile Ile Ala Ile Lys Glu Ala Tyr Gln Arg
        115                 120                 125

Leu Phe Asp Arg Ser Leu Glu Ser Asp Val Lys Gly Asp Thr Ser Gly
    130                 135                 140

Asn Leu Lys Lys Ile Leu Val Ser Leu Leu Gln Ala Asn Arg Asn Glu
145                 150                 155                 160

Gly Asp Asp Val Asp Lys Asp Leu Ala Gly Gln Asp Ala Lys Asp Leu
                165                 170                 175

Tyr Asp Ala Gly Glu Gly Arg Trp Gly Thr Asp Glu Leu Ala Phe Asn
            180                 185                 190

Glu Val Leu Ala Lys Arg Ser Tyr Lys Gln Leu Arg Ala Thr Phe Gln
        195                 200                 205

Ala Tyr Gln Ile Leu Ile Gly Lys Asp Ile Glu Glu Ala Ile Glu Glu
    210                 215                 220

Glu Thr Ser Gly Asp Leu Gln Lys Ala Tyr Leu Thr Leu Val Arg Cys
225                 230                 235                 240

Ala Gln Asp Cys Glu Asp Tyr Phe Ala Glu Arg Leu Tyr Lys Ser Met
                245                 250                 255

Lys Gly Ala Gly Thr Asp Glu Glu Thr Leu Ile Arg Ile Val Val Thr
            260                 265                 270

Arg Ala Glu Val Asp Leu Gln Gly Ile Lys Ala Lys Phe Gln Glu Lys
        275                 280                 285

Tyr Gln Lys Ser Leu Ser Asp Met Val Arg Ser Asp Thr Ser Gly Asp
    290                 295                 300

Phe Arg Lys Leu Leu Val Ala Leu Leu His
305                 310

<210>  14
<211>  233
<212>  PRT
<213>  Homo sapiens

<400>  14

Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe
            20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro
    50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230

<210>  15
<211>  157
<212>  PRT
<213>  Homo sapiens

<400>  15

<210>  16
<211>  235
<212>  PRT
<213>  Homo sapiens

<400>  16

Met Ser Thr Glu Ser Met Ile Arg Asp Val Glu Leu Ala Glu Glu Ala
1               5                   10                  15

Leu Pro Gln Lys Met Gly Gly Phe Gln Asn Ser Arg Arg Cys Leu Cys
            20                  25                  30

Leu Ser Leu Phe Ser Phe Leu Leu Val Ala Gly Ala Thr Thr Leu Phe
        35                  40                  45

Cys Leu Leu Asn Phe Gly Val Ile Gly Pro Gln Arg Asp Glu Lys Phe
    50                  55                  60

Pro Asn Gly Leu Pro Leu Ile Ser Ser Met Ala Gln Thr Leu Thr Leu
65                  70                  75                  80

Arg Ser Ser Ser Gln Asn Ser Ser Asp Lys Pro Val Ala His Val Val
                85                  90                  95

Ala Asn His Gln Val Glu Glu Gln Leu Glu Trp Leu Ser Gln Arg Ala
            100                 105                 110

Asn Ala Leu Leu Ala Asn Gly Met Asp Leu Lys Asp Asn Gln Leu Val
        115                 120                 125

Val Pro Ala Asp Gly Leu Tyr Leu Val Tyr Ser Gln Val Leu Phe Lys
    130                 135                 140

Gly Gln Gly Cys Pro Asp Tyr Val Leu Leu Thr His Thr Val Ser Arg
145                 150                 155                 160

Phe Ala Ile Ser Tyr Gln Glu Lys Val Asn Leu Leu Ser Ala Val Lys
                165                 170                 175

Ser Pro Cys Pro Lys Asp Thr Pro Glu Gly Ala Glu Leu Lys Pro Trp
            180                 185                 190

Tyr Glu Pro Ile Tyr Leu Gly Gly Val Phe Gln Leu Glu Lys Gly Asp
        195                 200                 205

Gln Leu Ser Ala Glu Val Asn Leu Pro Lys Tyr Leu Asp Phe Ala Glu
    210                 215                 220

Ser Gly Gln Val Tyr Phe Gly Val Ile Ala Leu
225                 230                 235

<210> 17
<211> 157
<212> PRT
<213> Homo sapiens

<400> 17

Met Leu Arg Ser Ser Ser Gln Asn Ser Ser Asp Lys Pro Val Ala His
1               5                   10                  15

Val Val Ala Asn His Gln Val Glu Glu Gln Leu Glu Trp Leu Ser Gln
            20                  25                  30

Arg Ala Asn Ala Leu Leu Ala Asn Gly Met Asp Leu Lys Asp Asn Gln
        35                  40                  45

Leu Val Val Pro Ala Asp Gly Leu Tyr Leu Val Tyr Ser Gln Val Leu
    50                  55                  60

Phe Lys Gly Gln Gly Cys Pro Asp Tyr Val Leu Leu Thr His Thr Val
65                  70                  75                  80

Ser Arg Phe Ala Ile Ser Tyr Gln Glu Lys Val Asn Leu Leu Ser Ala
                85                  90                  95

Val Lys Ser Pro Cys Pro Lys Asp Thr Pro Glu Gly Ala Glu Leu Lys
            100                 105                 110

Pro Trp Tyr Glu Pro Ile Tyr Leu Gly Gly Val Phe Gln Leu Glu Lys
        115                 120                 125

Gly Asp Gln Leu Ser Ala Glu Val Asn Leu Pro Lys Tyr Leu Asp Phe
    130                 135                 140

Ala Glu Ser Gly Gln Val Tyr Phe Gly Val Ile Ala Leu
145                 150                 155

## Description

### Related Applications

The present invention claims the benefit of priority of U.S. Provisional Application Number 62/157,395, filed May 5, 2015, the contents of which is hereby incorporated by reference in its entirety.

### Incorporation by Reference

The content of the following submission on ASCII text file is incorporated herein by reference in its entirety: a computer readable form (CRF) of the Sequence Listing (file name: 48516-501001WO_SEQLIST.txt, date recorded: May 5, 2016, size: 45,056 bytes).

### Field

The invention relates to immunotherapeutic compositions and methods for treating cancer, specifically solid tumours and cells of the tumour microenvironment.

### Background

A strategy for generating new anti-cancer therapeutics involves identifying differences between normal, healthy cells and tumour cells and cells of the tumour microenvironment. The aim is to exploit those differences to target therapeutics to tumour cells and cells of the tumour microenvironment, leaving the normal healthy cells unaffected.

Differences have been identified between normal, healthy cells and tumour cells and cells of the tumour microenvironment in the composition of phospholipids in plasma membranes. Certain phospholipids which are largely absent from the outer leaflet of the plasma membrane of normal, healthy cells become exposed on the outer leaflet of tumour cells and other cells of the tumour microenvironment.

An example of a phospholipid which is usually absent from the outer plasma membrane leaflet is phosphatidyl serine (PS). However, in tumour cells and cells of the tumour microenvironment PS is surface exposed. PS surface exposure on tumour cells suppresses the body's immune response to those cells. This effect of PS is accomplished via signaling to macrophages to engulf the cells where PS is located on the outer surface of the plasma membrane. The macrophage engulf a cell, rather than release inflammatory cytokines and other signalling agents, prevents a large scale immune response. Hence, this exposure inhibits inflammatory cytokine signalling from macrophages and provides one avenue by which the tumor microenvironment promotes immunosuppression.

Annexins are proteins which bind phospholipids. Annexin A5 has very high binding affinity for PS, thereby offering a potential tumour targeting molecule. A molecule such as the Fc fragment of an Ig linked to an annexin A5 could offer a conjugate which masks PS and so masks its immunosuppressive effect and provides an immunostimulatory effect. However, annexin A5 is rapidly internalised after binding to surface exposed PS limiting the effect of such a therapeutic strategy.

### Summary of the Invention

The present invention relates to an anti-cancer conjugate comprising an annexin which masks an immunosuppressive effect of a surface exposed phospholipid on a tumour cell or a cell of the tumour microenvironment, causes immunostimulation at the tumour site and persists at the outer surface of a cell at the tumour site to concentrate and/or prolong these effects in comparison with previously proposed anti-cancer therapeutics involving annexin. Furthermore, a single conjugate of the invention can provide the masking of the immunosuppressive effect of a phospholipid and immunostimulation.

The invention provides a conjugate comprising a non-internalising annexin capable of binding to at least one phospholipid and an immunostimulatory agent.

The invention further provides a polypeptide or nucleotide sequence encoding the conjugate of the invention.

The invention also provides the conjugate, polypeptide or nucleotide sequence disclosed herein for use in a method of medical treatment.

The invention further provides a method of treating cancer comprising administering to a patient in need thereof an effective amount of a conjugate comprising a non-internalising annexin capable of binding to at least one phospholipid and an immunostimulatory agent. The invention also provides the conjugate, polypeptide or nucleotide sequence described herein for use in a method of treating cancer.

Advantageously, the phospholipid to which the conjugate of the invention can bind is selected from phosphatidylserine (PS), phosphatidyl ethanolamine (PE), phosphatidyl inositol (PI) and its phosphoinositide derivatives (PIP, PIP2, PIP3), and preferably PS.

Preferably, the non-internalising annexin is annexin A5. Suitably, one or more amino acids selected from polar amino acids His, Glu, Gln, Asp, Asn, Arg and Lys in the helices IA, ID, IIA, IID, IIIC, IIID and IVE and in the stretches connecting helices IC and ID, IIE and IIIA, IIIC and IIID, IIID and IIIE, and IVA and IVB of annexin are replaced by non-polar amino acids to provide non-internalising annexin. Preferably, the non-internalising annexin comprises at least a portion of SEQ ID NO:1 for annexin A5 or the corresponding sequences for other annexins. Optionally, the one or more amino acids are located at positions 16-29, 59-74, 88-102, 135-145, 156-169, 202-231, 259-266 and 305-317 of SEQ ID NO:1 for annexin A5, or the corresponding sequences for other annexins.

Preferably, the non-internalising annexin comprises SEQ ID NO:1 for annexin A5 or the corresponding sequences for other annexins, wherein one or more amino acids selected from Glu, Gln, Asp, Asn, Arg, Lys and His at positions 16-29, 59-74, 88-102, 135-145, 156-169, 202-231, 259-266 and 305-317, or the corresponding sequences for other annexins, are replaced by Gly, Ala, Val, Ile, Leu, Ser, Thr, Met, Pro, Phe, or Tyr. Suitably, at least two of said polar amino acids are replaced by non-polar amino acids, preferably at least 3, 4, 5 or 6 of said polar amino acids are replaced by non-polar amino acids.

Suitably, the annexin is capable of binding to the phospholipid with a dissociation constant of about 10⁻⁶ M or less, preferably 10⁻⁷ M or less, more preferably 10⁻⁸ M or less, even more preferably 10⁻⁹ M or less.

Preferably, the immunostimulatory agent promotes an inflammatory response.

The immunostimulatory agent may be a nanoparticle such as a fullerene, carbon nanotube, dendrimer or other synthetically constructed supramolecular structure. For example, the nanoparticle may be a C60 nanoparticle, such as polyhydroxy-C60 (poly-C60) or N-ethyl-polyamino-C60 (nepo-C60).

Suitably, the immunostimulatory agent is selected from TNFα, IL1α, IL1β, IL2, IL4, IL6, IL8, IL10, IL12, IL15, IL17A, IFNγ, GM-CSF (CSF2), M-CSF (CSF1), G-CSF (CSF 3), or other applicable cytokines or chemokines, an immunoglobulin, and fragments, monomers, multimers, variants, muteins, post-translationally modified versions thereof and mixtures thereof.

Preferably, the immunostimulatory agent is a fragment crystallizable (Fc) region of an immunoglobulin, monomer or fragment thereof, more preferably IgG, even more preferably human IgG1 or IgG3.

Preferably, the immunostimulatory agent is TNF-α.

Advantageously, the non-internalising annexin is linked to the immunostimulatory agent via a linker. Preferably, the non-internalising annexin is linked to the immunostimulatory agent via the N- or C- terminus, preferably via the N- terminus. Suitably, the conjugate comprises a plurality of immunostimulatory agents and optionally a plurality of linkers.

Alternately, the non-internalizing annexin is linked to a liposome containing one or more of the immunostimulatory agents.

Optionally cancer treatable by a conjugate of the present invention is a solid tumour. The tumour may be selected from breast, triple negative breast, ovarian , prostate, castrate-resistant prostate, pancreatic, bladder, bone, head and neck, lung, liver, thyroid, esophageal, stomach, intestinal, brain, glioblastoma.

### Brief Description of the Drawings

Fig. 1 depicts the amino acid sequence for wild-type annexin A5 (SEQ ID NO: 1).

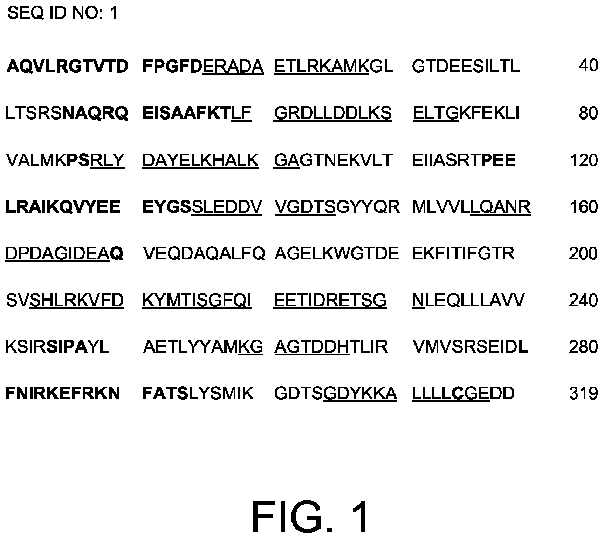

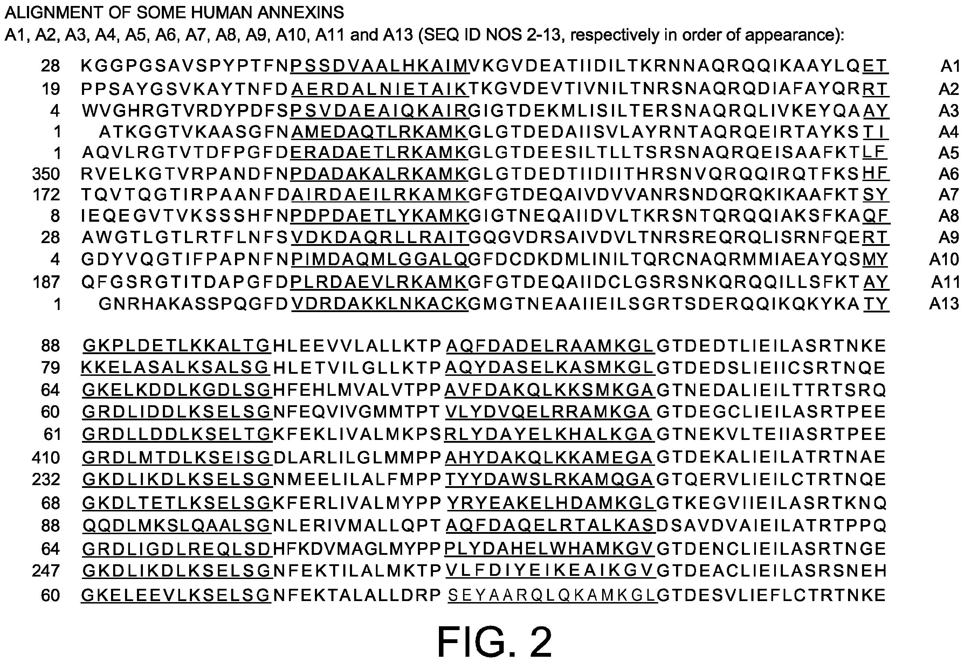

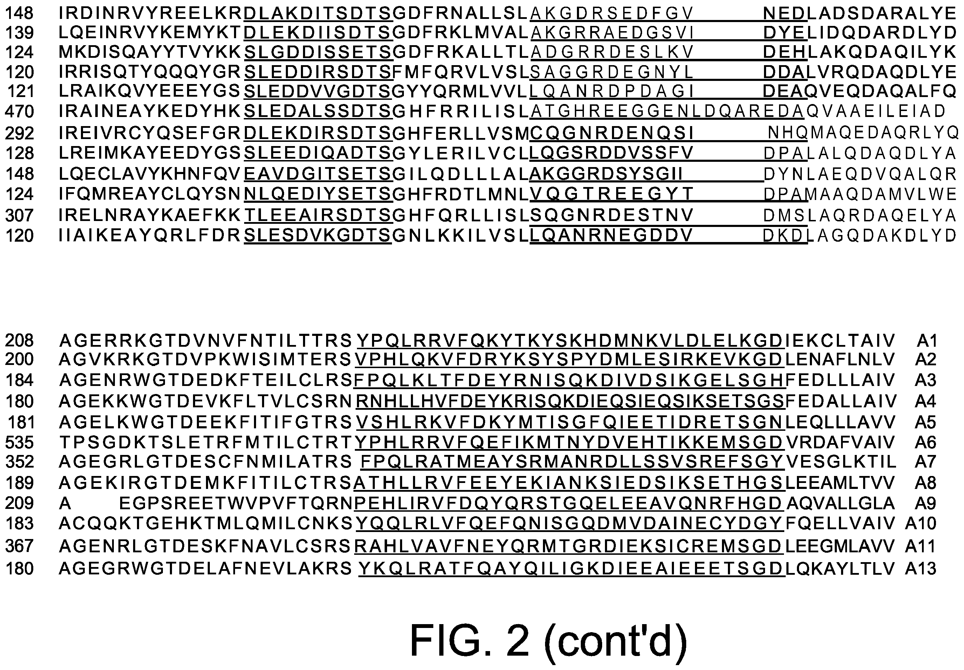

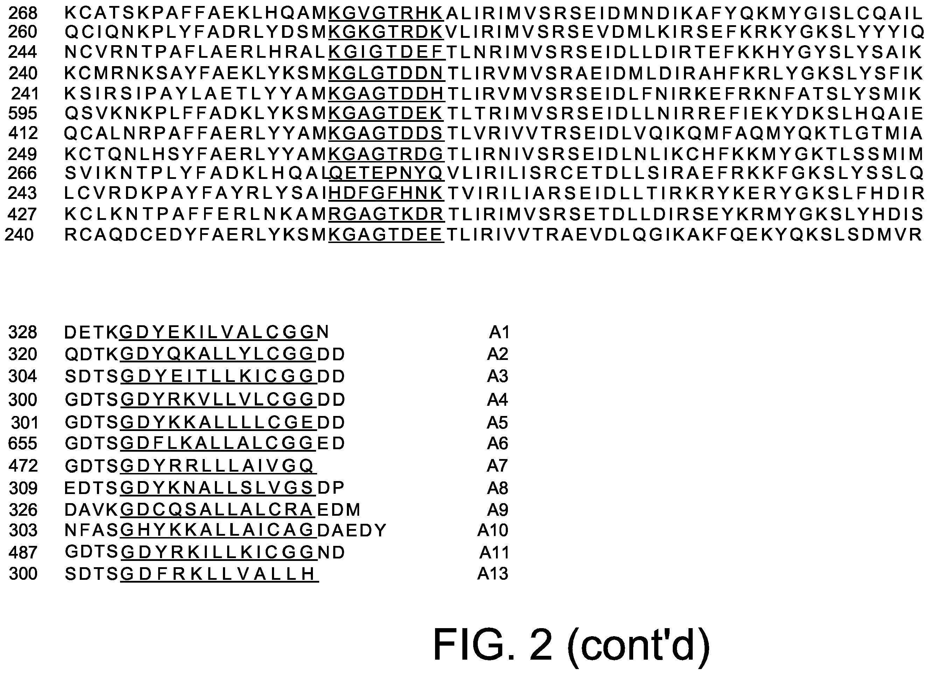

Fig. 2 depicts an amino acid sequence alignment between human annexins A1, A2, A3, A4, A5, A6, A7, A8, A9, A10, A11 and A13 (SEQ ID NOS: 2-13). Amino acid positions 16-29, 59-74, 88-102, 135-145, 156-169, 202-231, 259-266, and 305-317 of annexin A5 and corresponding positions of other annexins are underlined in Fig. 1. One or more of these amino acids may be replaced to provide a non-internalising annexin variant. Amino acids located at the concave side of the annexin molecule are represented by italic amino acid symbols in Fig. 1. Amino acid positions 1-15, 46-58, 86-87, 118-134, 170, 245-248 and 280-294 of annexin A5 and the corresponding stretches in other annexins are the bold italic positions in Fig. 1.

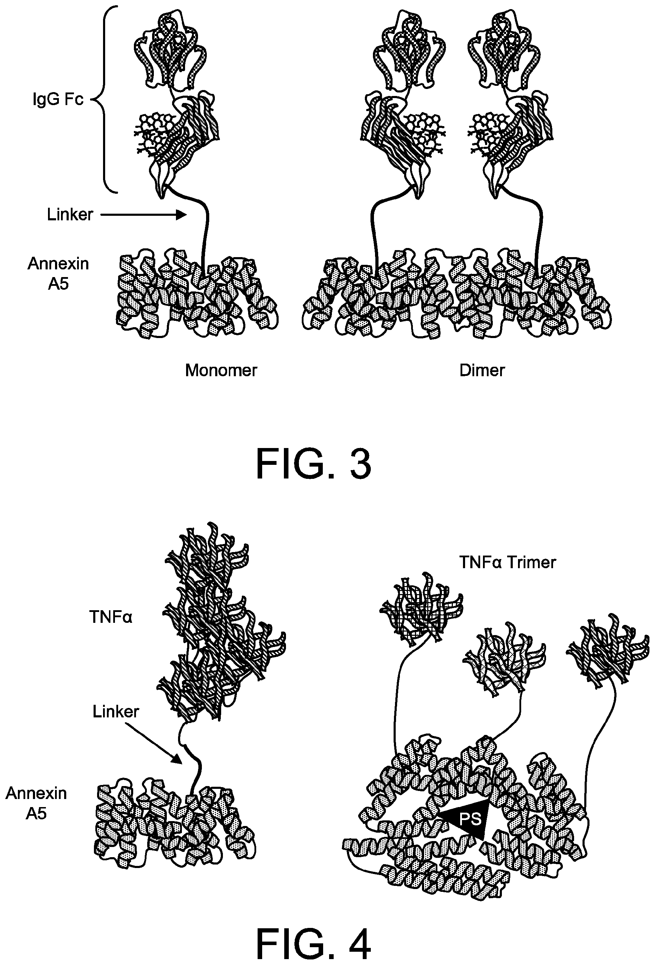

Fig. 3 depicts a conjugate comprising a non-internalising annexin A5 and IgG Fc.
Fig. 4 depicts a conjugate comprising a non-internalising annexin A5 and TNF-α.
Fig. 5 depicts amino acid sequences for TNF-α SEQ ID NOS: 14-17.

### Detailed Description

The invention provides a conjugate comprising a non-internalising annexin capable of binding to at least one phospholipid and an immunostimulatory agent.

As used herein, a "conjugate" is a molecule comprising at least two parts associated such that the parts of the molecule remains associated if transported to a target. Conjugates include fusion proteins linked to each other via their polypeptide structure, through genetic expression of a DNA molecule encoding these proteins, directly synthesised proteins and coupled proteins in which pre-formed sequences are associated by cross-linking agents or associations, such as aggregates of the parts of the molecule.

An "annexin" is a protein characterised by its ability to bind phospholipid, particularly anionic phospholipid, in a calcium dependent manner. Annexins are also characterised by a 70 amino acid repeat sequence called an annexin repeat. The basic structure of an annexin comprises two major domains. The first is located at the COOH terminal and is called the "core" region. The second is located at the NH₂ terminal and is called the "head" region. The core region consists of an alpha helical disk. The convex side of this disk has type 2 calcium-binding sites important for allowing interaction with the phospholipids at the plasma membrane.

As used herein, "non-internalising" in the context of a non-internalising annexin means an annexin that, once bound to phospholipid, will remain on the cellular surface for a longer period of time compared to a wild-type annexin. A non-internalising annexin may have reduced ability to form an annexin trimer, or reduced ability for an annexin trimer to form a lattice with other annexin trimers or both. In the case of annexin A5, a two-dimensional lattice is not formed that would otherwise bend the plasma membrane nanomechanically to elicit budding and endocytic vesicle formation leading to pinocytosis. A non-internalising annexin typically has one or more amino acids replaced in helices IA, ID, IIA, IID, IIIC, IIID and IVE and in the stretches connecting helices IC and ID, IIE and IIIA, IIIC and IIID, IIID and IIIE, and IVA and IVB.

By "immunostimulatory agent" is meant an agent that stimulates the immune system by inducing activation or increasing activity of any of its components. Preferably, the agent promotes an inflammatory response.

As used herein, "post-translationally modified" or a "post-translational modification" includes, but is not limited to, phosphorylation, acetylation, methylation, ubiquitination, sumoylation, hydroxylation, citrullination (deimination), deamidation, oxidation, reduction, or glycosylation. The modification can occur either through biological processes (such as enzymatically in vivo or in vitro) or through synthetic processes (such as a chemical reaction in vitro).

As used herein, "immunoglobulin" (also known as "antibody") is a protein produced by plasma cells that is used by the immune system to identify and neutralize pathogens such as bacteria and viruses via a specific interaction with an antigen. The protein is typically made of basic structural units-each with two large heavy chains and two small light chains. The fragment crystallisable (or Fc) region of an immunoglobulin is composed of two heavy chains that contribute two or three constant domains depending on the class of the antibody. The Fc region ensures that each antibody generates an appropriate immune response for a given antigen.

By "cancer" is meant a family of diseases that involve abnormal cell growth with the potential to invade or spread to other parts of the body. A tumour is a group of cells that are transformed and grow without normal cell regulation. Reference to treating cancer or treating a tumour can include cells of a tumour mass and cells of the tumour microenvironment, such as tumour vasculature and stromal cells (e.g. fibroblasts and immune cells) for example.

Phospholipids are a class of lipids having a hydrophilic phosphate "head" and two hydrophobic "tails" usually consisting of 2 long fatty acid hydrocarbon chains. Phospholipids are a major component of all cell membranes as they can form lipid bilayers. A lipid bilayer, also known as a phospholipid bilayer, is a sheet of lipids two molecules thick, arranged so that the hydrophilic heads point "out" to the water on either side of the bilayer and the hydrophobic tails point "in" to the core of the bilayer. This arrangement results in two "leaflets" assembled due to the hydrophobic effect.

In many naturally occurring bilayers, the compositions of the inner and outer membrane leaflets are different.

The phospholipids phosphotidyl serine (PS) and phosphotidyl inositol (PI) and its derivatives (PIP, PIP2, PIP3) are largely absent from the surface of healthy mammalian cells under normal conditions with 96% or more found on the inner leaflet of the plasma membrane. PE is mostly in the inner leaflet of resting mammalian cells under normal conditions, with approximately 20% present in the outer leaflet.

Sequestration of PS to the cytosolic leaflet is driven by ATP-hydrolysis and dependent on an aminophospholipid translocase (APLT), whose actions under certain conditions such as apoptosis are countered by a "scramblase" activity that disrupts the membrane asymmetry of PS. Other conditions where PS is detected on the external cell surface include aging erythrocytes, activated platelets and macrophages, and necrotic cells. Disruption of PS asymmetry also appears to be a consequence of hypoxia and oxidative stress as demonstrated *in vitro.*

Cell surface exposure of PS has several roles, including promoting coagulation in damaged blood vessels and mediating attachment of T-cells to thrombin-activated endothelial cells. It also plays a role in suppressing an auto-immune response against self-antigens by signalling macrophages to engulf cells on which PS is surface exposed while modulating cytokine production.

Clearance of apoptotic cells by phagocytes, termed efferocytosis, plays a pivotal role in normal homeostasis. Efferocytosis is important to promote an anti-inflammatory phenotypic change in macrophages by causing the release of anti-inflammatory cytokines, antiproteinases and growth factors. It has also been shown to reduce proinflammatory mediators such as TNF-alpha. Phosphatidylserine (PS) is involved in one pathway to initiate efferocytosis. Apoptotic cells lose phospholipid asymmetry of the plasma membrane, and PS is exposed on the outer leaflet of the lipid bilayer in the early phase of an apoptotic cell. Therefore, PS acts as signal for efferocytosis. In addition, PS-mediated efferocytosis has proven effects on macrophage function both *in vitro* and *in vivo.*

PS exposure on the cell surface is a characteristic of cancer cells, particularly solid tumour cells and cells associated with the tumour vasculature. Conditions of hypoxia and oxidative stress are also common in the tumour microenvironment.

Tumours avoid a full immunological assault by manipulating key "checkpoints" designed to prevent auto-immune disease. Blockading these "checkpoints" has included development and FDA-approval of ipilimumab, an antibody targeting CTLA-4 to inhibit downregulation of T-cell activation. It has also included development of antibodies to disrupt the PD-1/PD-L1 ligand-receptor complex. Yet, these therapies do not localize their activity to the tumours alone and affect the overall immune system.

While the role of surface-exposed PS appears to be key in helping suppress the immune response against dead and dying cells, PS exposure on living tumour cells and their vasculatures provides one avenue by which the tumour microenvironment promotes immunosuppression.

A conjugate according to the invention comprises a non internalising annexin capable of binding to at least one phospholipid. Preferably the phospholipid is not found on the surface of healthy, normal mammalian cells. Preferably the phospholipid is expressed on the surface of cancer cells only, or is expressed in a different amount on the surface of cancer cells compared to healthy, normal mammalian cells.

Anionic phospholipids appear on the surface of cancer cells, particularly tumour cells and cells of the tumour microenvironment and certain aging or apoptotic cells. Anionic phospholipids include phosphatidyl serine (PS), phosphatidic acid (PA), phosphatidyl glycerol (PG), cardiolipin (CL), phosphatidyl inositol (PI) and its phosphoinositide derivatives (PIP, PIP2, PIP3).

Neutral phospholipids include phosphatidyl ethanolamine (PE), phosphatidyl choline (PC) and sphingomyelin (SM).

Aminophospholipids are phospholipids that include within their structure at least a first primary amino group and occur in mammalian cell membranes. PS and PE are examples of aminophospholipids.

In particular, cancer cells can be characterised by cells displaying extracellular phosphatidylserine (PS), phosphatidyl inositol (PI) or its phosphoinositide derivatives (PIP, PIP2, PIP3); or by displaying a different quantity of extracellular phosphatidyl ethanolamine (PE) compared to healthy, normal mammalian cells.

Preferably, the cancer cells can be characterised by cells displaying extracellular anionic aminophospholipid. Preferably, the conjugate comprises a non-internalising annexin capable of binding to PS.

Annexin A5 (Annexin V) is a natural high-affinity PS-binding protein (Kd = 100 pM at the physiological ionic strength of 150 mM). It is a 36 kD non-glycosylated protein containing four domains, only one of which (Domain I) binds PS in a Ca2+ ion-dependent manner. Annexin A5 is present as a monomer when in solution, but once bound to PS on the membrane surface, it assembles into trimeric structures; in turn, each trimer interacts with other trimers in its proximity to form a two-dimensional lattice covering the PS-exposed surface. Trimerized annexin binding of PS has multiple roles, including inhibition of PS-triggered coagulation in blood vessels and repair of disrupted plasma membranes. A two-dimensional Annexin A5 lattice will inwardly bend the plasma membrane nanomechanically to elicit budding and endocytic vesicle formation leading to pinocytosis.

Human variants of Annexin A5 that do not internalize upon PS binding have been developed as an apoptosis imaging agent. Mutation of key amino acid residues responsible for salt bridge formation between Annexin A5 Domains I and III result in a variant that still binds PS but does not form a two-dimensional lattice and/or does not elicit inward bending of the plasma membrane. However, conjugation of this annexin to a therapeutic agent has not been contemplated because of predicted toxic side-effects.

Annexin A5 inhibits PS-mediated efferocytosis. This protein interferes with the recognition of apoptotic cells by macrophages, leading to attenuated efferocytosis. Furthermore, mutant annexin A5, which has lost binding affinity to PS, does not affect macrophage efferocytosis. Annexin A5 has been shown to attenuate macrophage efferocytosis *in vitro* and *in vivo,* alter the macrophage phenotype toward a more inflammatory one and result in an increase in the amount of inflammatory-associated disease.

A conjugate according to the invention can comprise a non-internalising annexin A1; annexin A10; annexin A11; annexin A13; annexin A2; annexin A3; annexin A4; annexin A5; annexin A6; annexin A7; annexin A8; annexin A8L1; annexin A8L2; or annexin A9. Preferably the annexin is annexin A5.

Wild type Annexin A5 can have the amino acid sequence of SEQ ID No. 1 (see Fig. 1). The present invention embraces conjugates comprising non-internalising variants of the annexins listed above. Reference is made to the amino acid sequence and the positions of annexin A5, but what applies to annexin A5 also applies to the other annexins, especially human annexins, by choosing the corresponding position found with the alignment of any annexins (see alignment of human annexins A1 to A11 and A13 in Fig. 2).

Preferably, the annexin is capable of binding the phospholipid with a dissociation constant of about 10⁻⁶ M or less, about 10⁻⁷ M or less, about 10⁻⁸ M or less, or about 10⁻⁹ M or less, preferably about 10⁻¹⁰ M or less. Preferably, the annexin is capable of binding the phospholipid with a dissociation constant of from about 10⁻⁸ M to about 10⁻¹⁰ M, preferably from about 20⁻⁹ M to about 10⁻¹⁰ M, preferably about 50⁻¹⁰ M.

A conjugate according to the invention comprises a non-internalising annexin. Non-internalising annexins are described in Ungethum et. al. (2011) the Journal of Biological Chemistry 286, 3, 1903-1910 (which is incorporated herein by reference) and typically have one or more amino acids replaced in helices IA, ID, IIA, IID, IIIC, IIID and IVE and in the stretches connecting helices IC and ID, HE and IIIA, IIIC and IIID, IIID and IIIE, and IVA and IVB. Preferably, one or more amino acids selected from polar amino acids His, Glu, Gln, Asp, Asn, Arg and Lys are replaced by non-polar amino acids. Suitably, the one or more amino acids are located at positions 16-29, 59-74, 88-102, 135-145, 156-169, 202-231, 259-266 and 305-317 of SEQ ID NO:1 for annexin A5, or the corresponding sequences for other annexins.

Preferably, one or more amino acids selected from Glu, Gln, Asp, Asn, Arg, Lys and His at positions 16-29, 59-74, 88-102, 135-145, 156-169, 202-231, 259-266 and 305-317 of SEQ ID NO:1 for annexin A5 or the corresponding sequences for other annexins are replaced by Gly, Ala, Val, Ile, Leu, Ser, Thr, Met, Pro, Phe, or Tyr.

A conjugate according to the invention preferably comprises a non-internalising annexin wherein at one or more, least 2, 3, 4, 5 or 6 of the polar amino acids are replaced by non-polar amino acids.

A conjugate according to the invention can comprise a non-internalising annexin having a cysteine residue at one or more amino acid positions selected from 1-15, 46-58, 86-87, 118-134, 170, 245-248 and 280-294 of annexin A5 and the corresponding stretches in other annexins, preferably at position 2.

Once bound to phospholipid, a conjugate according to the invention comprising a non-internalising annexin will remain on the cellular surface for a longer period of time compared to a wild-type annexin. The conjugate may provide improved and/or sustained masking of the immunosuppressive effects of PS exposed on the surface of cancer cells and the tumor vasculature, thus blocking a "checkpoint" utilised by the cancer, particularly a tumour and cells of the tumour microenvironment.

Suitably, the non-internalising annexin may have an impaired ability to form trimers. Suitably, the non-internalising annexin may have an impaired ability to form a two-dimensional network on the cellular surface. Preferably, the non-internalising annexin may have an impaired ability to form trimers and a two-dimensional network on the cellular surface.

A conjugate according to the invention comprises one or more immunostimulatory agents. Preferably, the immunostimulatory agent promotes an inflammatory response. The immunostimulatory agent can be selected from TNFα, IL1α, IL1β, IL2, IL4, IL6, IL8, IL10, IL12, IL15, IL17A, IFNγ, GM-CSF (CSF2), M-CSF (CSF1), G-CSF (CSF 3), an immunoglobulin, and fragments, monomers, multimers, variants, muteins, post-translationally modified versions thereof and mixtures thereof.

The immunostimulatory agent can be a fragment crystallizable (Fc) region of an immunoglobulin, monomer or fragment thereof. The immunoglobulin can be selected from IgG, IgM, IgA, and IgE, in particular their fragments. The immunoglobulin can be IgG. The IgG may be selected from IgG1, IgG2, IgG3 or IgG4, preferably IgG1 or IgG3, even more preferably IgG1. Conjugates according to the invention can comprise an Fc region of an immunoglobulin in the human or murine form, preferably human.

A conjugate according to the invention preferably comprises one or more human IgG1 Fc region, fragment, monomer, multimer, variant, mutein, post-translationally modified version thereof or mixture thereof.

A conjugate according to the invention can comprise an IgG Fc region with a modified amino acid sequence, or modified carbohydrates attached to the Fc region, to enhance complement dependent cytotoxicity (CDC), antibody dependent cell cytotoxicity (ADCC), and antibody dependent cellular phagocytosis (ADCP). For a review see Liu et. al. (2008) Immunological Reviews 222, 9-27 (which is incorporated herein by reference). For example, mutations of the IgG Fc region such as a double amino acid substitution Lys222Trp and Thr223Trp, or double substitution of Cys220Asp and Asp221Cys, or quadruple substitution of Cys220Asp and Asp221Cys and Lys222Trp and Thr223Trp, are some examples of mutations that enhance CDC. As another example, triple substitutions of Ser298Ala and Glu333Ala and Lys334Ala, or double amino acid substitution of Ser239Asp and Ile332Glu, enhance ADCC.

In this manner, a conjugate according to the invention can simultaneous shield PS from macrophage PS receptors and bind to macrophage Fcγ receptors. Such an interaction helps shift the polarization of tumour associated macrophages (TAMs) into the M1 phenotype and alter the cytokine balance within the tumour.

Modifications can also be undertaken to either enhance or reduce Fc binding to the neonatal Fc receptor (FcRn) in order to modify the conjugates' circulation times in vivo. For example, amino acid residues located in the interface between the IgG Fc constant domains Cγ2 and Cγ3 are involved in FcRn binding. As an example, modifications to one or more amino acids, such as Ile253, Ser254, Arg255, Lys288, Leu309, Ser415, His433, His435 and Tyr436 are predicted to reduce binding to human FcRn. Modifications to one or more amino acids such as Pro238, Thr256, Glu272, Val305, Thr307, His310, Gln311, Asp312, Lys317, Asp376, Ala378, Glu380, Glu382, Ser424 and Asn434 are predicted to enhance binding to human FcRn (see Table 4 of Martin et al. (2001) Molecular Cell 7, 867-877 [which is incorporated herein by reference]).

The immunostimulatory agent can be TNF-α. The term "TNF-α" is used according to its plain ordinary meaning in the art and refers to is a cell signaling protein (cytokine) involved in systemic inflammation and is one of the cytokines that make up the acute phase reaction. TNF-α includes modified proteins and homologs of the same. Human TNF-α may be a non-glycosylated soluble protein of 17 kDa and a length of 157 amino acids. Murine TNF-α is N-glycosylated. Homology with TNF-beta is approximately 30%. The 17 kDa form is produced by processing of a precursor protein of 233 amino acids. A transmembrane form of TNF-α of 26 kDa has also been described.

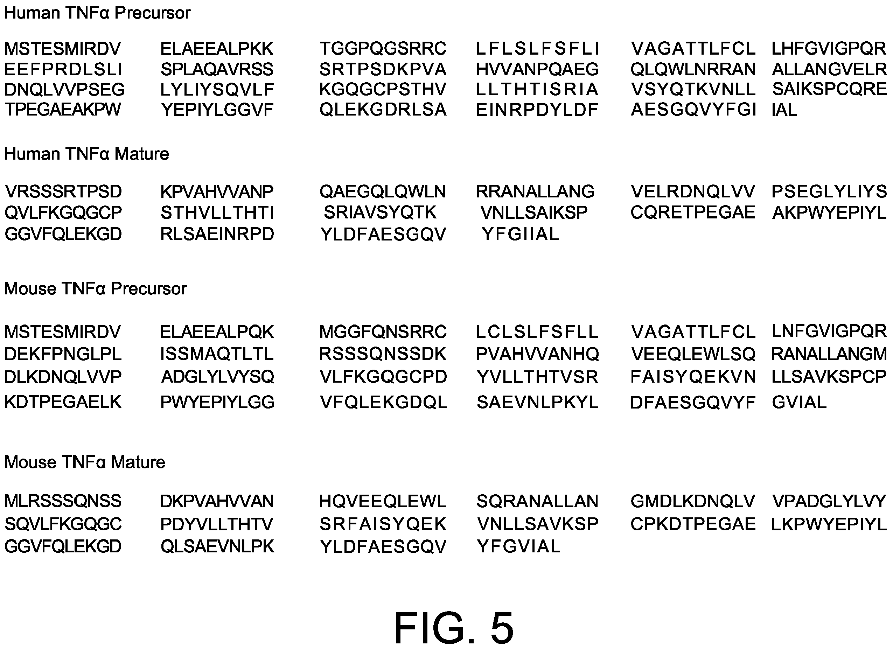

A conjugate according to the invention may comprises TNF-α in the soluble or transmembrane form, preferably the soluble form. Conjugates according to the invention can comprise TNF-α as a full protein, precursor or fragment thereof (as described in US 7,892,558, which is incorporated herein by reference). Conjugates according to the invention can comprise TNF-α in the human or murine form, preferably human. TNF-α can be post-translationally modified. A conjugate according to the invention can comprise glycosylated or non-glycosylated TNF-α. Preferably, the conjugate according to the invention comprises SEQ ID NO: 14, 15, 16 or 17 (see Fig. 5).

A conjugate according to the invention can comprise TNF-α variants. Variants can be modified genetically (muteins) or chemically to be more or less toxic. For example, mutations Ala84 to Val and Val91 to Ala reduce the cytotoxic activity of the factor almost completely. Deletion of 7 N-terminal amino acids and the replacement of Pro8Ser9Asp10 by ArgLysArg yields a mutated factor with an approximately 10-fold enhancement in antitumor activity, increased receptor binding (L-M cell assay) and reduced toxicity. Other TNF-α variants are described in Patents US 7,446,174; US 7,056,695; US 7,118,750; US 6,878,370; US 7,642,340; patent applications US20020110868A1, US20070172449A1 and US20070207961A1, all of which are incorporated herein by reference. Variants can be modified chemically with small molecules or with artificial (e.g. poly-ethyleneglycol) or natural (e.g. carbohydrates) polymers. TNF-α variants may improve annexin trimerization and thereby enhance toxicity.

A conjugate according to the invention preferably comprises one or more human TNF-α, fragment, monomer, multimer, variant, mutein, post-translationally modified version thereof or mixture thereof.

In this manner, a conjugate according to the invention can simultaneously shield PS from macrophage PS receptors and promote an inflammatory response.

The conjugate of the present invention provides improved and/or sustained immunostimulatory effects.

A conjugate according to the invention can comprise a non-internalising annexin and a plurality of immunostimulatory agents, preferably two or three immunostimulatory agents.

The non-internalising annexin can be linked to the immunostimulatory agent via a linker. Moieties can be linked through conjugation of the N- or C-terminus of the annexin protein, through conjugation of the amines (lysine or arginine), sulfhydryls (e.g. cysteine), carboxyls (e.g. c-terminus), carbohydrates (e.g. oxidized sugars) or non-selectively (e.g. photoreactive crosslinking agents, formaldehyde, glutaraldehyde). Linkage can occur to amino acids found in nature or artificially constructed amino acids introduced into the sequence of the non-internalizing annexin or the immunostimulatory agent.

Linkers can be peptides, proteins, carbohydrates, nucleic acids, morpholinos (synthetic oligonucleotide analogues containing morpholino-phosphorodiimidate chains instead of deoxyribose-phosphodiester chains), peptide nucleic acids (synthetic oligonucleotide analogues containing N-aminoethyl-glycine chains instead of deoxyribose-phosphodiester chains, PNA).

The linker preferably comprises a peptide that promotes product stability and in vivo half-life. Examples of such linkers are Glycine-Serine linkers: (GGGGS)n; helix-forming peptide linkers: A(EAAAK)nA (Arai et al. 2001 Protein Engineering); a sequence of Pro, Ala and/or Ser amino acids: (PASn). Other peptide linkers are proteins or protein fragments that function as a spacer. In one example, "tags" can be used to link the annexin to the immunostimulatory agent. These tags can be a histidine tag (e.g. 6X His) or larger (e.g. c-myc, chitin binding protein (CBP), maltose binding protein (MBP), and glutathione-S-transferase (GST), human influenza hemagglutinin (HA) or the FLAG tag DYKDDDDK)). Linkers can be N-tails of other annexins, preferably an annexin selected from any of the annexins in Figure 2.

Preferably, the linker can be a peptide linker comprising 1-50 amino acids, preferably about 1-10, 1-20, 1-30, 1-40, 1-50; about 20-30, 20-40, 20-50; about 30-40, 30-50; about 40-50 amino acids; preferably 1, 5, 10, 15, 20, 25, 30, 35, 40, 45, 50 amino acids.

Linkers can be non-cleavable or cleavable (e.g. cleavage using thiols, acids, bases, hydroxylamines or periodates). Linkers consisting of a crosslinking reagent can be homo-bi-functional (e.g. amine to amine) or hetero-bi-functional (e.g. amine to sulfhydryl). These crosslinkers can have lengths ranging from zero angstroms (e.g. formaldehyde), a defined length of, for example, 95.2 angstroms (SM(PEG)n NHS-PEG-Maleimide Crosslinkers from Life Technologies) and greater (e.g. glutaraldehyde). Linkers can be a thioether (e.g. succinimidyl-4-(N-maleimidomethyl) cyclohexane-1-carboxylate), known also by its name TAP (Tumour-Activated Prodrug) linker.

The non-internalising annexin can be linked to the immunostimulatory agent covalently or non-covalently. Linkers can be streptavidin/avidin and biotin combination, combination of complementary DNA and RNA oligonucleotides, complementary DNA and RNA analogs such as morpholinos (synthetic oligonucleotide analogues containing morpholino-phosphorodiimidate chains instead of deoxyribose-phosphodiester chains), peptide nucleic acids (synthetic oligonucleotide analogues containing N-aminoethyl-glycine chains instead of deoxyribose-phosphodiester chains, PNA) and aptamers (specifically binding oligonucleotides or oligopeptides), the antibody and hapten combination, and the receptor and ligand combination.

Preferably, the linker may be from 0 to about 200 angstroms in length; preferably from about 0-100; about 1-10, 1-20, 1-30, 1-40, 1-50, 1-60, 1-70, 1-80, 1-90 or 1-100 angstroms in length; about 10-20, 10-30, 10-40, 10-50, 10-60, 10-70, 10-80, 10-90 or 10-100 angstroms in length; about 20-30, 20-40, 20-50, 20-60, 20-70, 20-80, 20-90 or 20-100 angstroms in length; about 30-40, 30-50, 30-60, 30-70, 30-80, 30-90 or 30-100 angstroms in length; about 40-50, 40-60, 40-70, 40-80, 40-90 or 40-100 angstroms in length; about 50-60, 50-70, 50-80, 50-90 or 50-100 angstroms in length; about 60-70, 60-80, 60-90 or 60-100 angstroms in length; about 70-80, 70-90 or 70-100 angstroms in length; about 80-90 or 80-100 angstroms in length; or about 90-100 angstroms in length; preferably about 1, 5, 10, 15, 20, 25, 30, 35, 40, 45, 50, 55, 60, 65, 70, 75, 80, 85, 90, 95 or 100 angstroms in length.

Preferably, the linker may be about 17, about 24, about 32, about 39, about 53, or about 95 angstroms in length.

Preferably the non-internalising annexin may be linked to the immunostimulatory agent via the N- or C- terminus of the annexin, preferably via the N- terminus. Preferably the linkage is covalent.

A conjugate according to the invention can comprise a plurality of linkers. Where the conjugate comprises a plurality of immunostimulatory agents, one or more linkers can link each immunostimulatory agent to the annexin or to each other.

Phospholipid bilayers may further form liposomes or vesicles. Liposomes are bubble like structures formed from the lipid bilayers. Liposomes may contain small amounts of other molecules (e.g., immunostimulatory agents). Liposomes can vary in range of size from micrometers to tens of micrometers. Liposomes may comprise any of the phospholipids disclosed herein.

A conjugate according to the invention may include a liposome. In some embodiments, the the annexin is conjugated to a liposome. In some embodiments, the liposome can include other molecules (e.g. immunostimulatory agents).

The invention relates to a polypeptide comprising a non-internalising annexin and an immunostimulatory agent as defined herein and a nucleotide sequence encoding such a polypeptide. Preferably, the polypeptide comprises a linker as defined herein.

The present invention provides conjugates for use in medical treatment. In particular, a conjugate of the invention can be used to treat cancer. The invention is particularly useful in treating cancer characterised by cells displaying extracellular PS, PI, PIP, PIP2 or PIP3; or by displaying a different quantity of extracellular PE compared to healthy, normal mammalian cells. Preferably, the cancer cells display greater than 20% of PE in the outer leaflet of the cellular membrane. Preferably, the cancer cells display greater than 30% of PE in the outer leaflet of the cellular membrane.

Preferably, the invention provides conjugates for treating tumour cells and cells of the tumour microenvironment. Cells of the tumour microenvironment include tumour vasculature and stromal cells (e.g. fibroblasts and immune cells) that support the tumour. Tumour cells and cells of the tumour microenvironment display PS, PI, PIP, PIP2 or PIP3 abnormally in their outer leaflet, preferably PS; or display a different quantity of extracellular PE compared to healthy, normal mammalian cells. Suitably, the tumour is selected from breast, triple negative breast, ovarian , prostate, castrate-resistant prostate, pancreatic, bladder, bone, head and neck, lung, liver, thyroid, esophageal, stomach, intestinal, brain, glioblastoma.

Healthy, normal mammalian cells can be distinguished from cancer cells based on the presence of phosphatidylserine (PS), phosphatidyl inositol (PI) or its phosphoinositide derivatives (PIP, PIP2, PIP3) in the outer leaflet of the membrane bilayer; or a different quantity of phosphatidyl ethanolamine (PE) in the outer leaflet of the membrane bilayer. PS, for example, can be detected on the extracellular surface of cells using radiolabelled or fluorescently labelled annexin A5 using flow cytometry of disaggregated cells, fluorescent microscopy of tissue sections, or in vivo imaging of PS exposed cells with SPECT imaging. Another technique is to add a conjugation agent to modify any PS that is exposed on the outer leaflet. Cells are then disrupted to quantify modified vs. unmodified PS. Unmodified PS would represent PS on the inner leaflet while modified PS would represent PS on the outer leaflet.

Methods of treating cancer involve administering a therapeutically effective amount of the conjugate according to the invention to an animal or patient in need of such treatment, optionally with one or more pharmaceutically acceptable excipient.

Suitably, the cancer can be treated by administering only a single therapeutic agent. For example, if the conjugate according to the invention is administered intravenously, the treatment can consist of only a single procedure for the patient.

In another embodiment, the invention provides a method of preparing a conjugate according to the invention comprising conjugating a non-internalising annexin having a cysteine residue at one or more amino acid positions selected from 1-15, 46-58, 86-87, 118-134, 170, 245-248 and 280-294 of annexin A5 and the corresponding stretches in other annexins, preferably at position 2, to an immunostimulatory agent; optionally via a linker as described herein.

The invention provides a method of preparing a fusion protein or nucleic acid as described herein using methods known in the art.

### Examples

### Non-internalising annexin A5 IgG Fc

Non-internalizing annexin A5 is manufactured in a microbial system, purified by chromatographic methods and then chemically conjugated at the N-terminal cysteine site to IgG Fc acquired commercially.

The gene construct encoding a polypeptide chain comprising a non-internalizing annexin A5 fused to Fc is transfected into microbial and mammalian expression systems.

### Non-internalising annexin A5 TNF- α

Non-internalizing annexin A5 is manufactured in a microbial system, purified by chromatographic methods and then chemically conjugated at the N-terminal cysteine site to TNF-α acquired commercially.

The gene construct encoding a polypeptide chain comprising a non-internalizing annexin A5 fused to TNF-α is transfected into microbial and mammalian expression systems.

### Product characterisation

Conjugates are tested for degradation using SDS-PAGE, product aggregation using size exclusion chromatography (SEC), product identity and stability using mass spectrometry. These are standard techniques. Binding potency to PS is evaluated using assays described in the literature.

Non cell-based assay format: PS is dissolved in n-hexane to a concentration of 50 µg/mL and applied to a 96-well plate in 100 µL aliquots. After evaporation of the solvent in a fume hood, the plates are blocked with a blocking agent and then incubated with serial dilutions of annexin A5 (AnxA5) conjugates for 2 hours at 25°C before being rinsed with wash buffer (Tris buffered saline with 0.1% Tween 20) and incubated with an appropriate secondary antibody conjugated to horseradish peroxidase for 30 minutes at 25°C (goat anti-mouse IgG for AnxA5-Fc; anti-TNFa and then goat anti-mouse IgG for AnxA-TNFα). Upon further washing, the plates are developed with TMB substrate and read at 450 nm wavelength.

Cell-based assay format: PS exposure is induced on the surface of cultured cells, without causing apoptosis, by treating cells either with 200 µM H₂O₂ in serum-free media for 1 hour at 37°C or 20 pM docetaxel in media for 24 hours at 37°C. Cells are washed with PBS, incubated with AnxA5 conjugates in serum-free media for 1 hour at 25°C before being washed again and then fixed with 4% (v/v) paraformaldehyde in PBS for 15 minutes. Binding of AnxA5 or the conjugates to the induced cells is compared with untreated cells using an anti-Annexin A5 antibody (e.g. Abcam cat. # ab14196) and a suitable secondary antibody conjugated to a fluorescent probe. The fixed cells can also be counterstained with DAPI to detect DNA in the nuclei.

Toxicity study: Using female BALB/c mice, groups of mice (n=3) receive 0, 0.3, 1, 3 and 10 mg/kg of AnxA5 conjugates on Day 1. Physical examination and survivability (at pre-treatment (-1), Day 1, Day 7); Body weights (on Days -14, -7, -1, 1, 7); Clinical observations (twice daily on days -7 to 7 plus pre-dose and 1, 6 h post-dose); Food and water consumption (on Day -7 to 7); Gross pathology and organ weights (including heart, liver, kidneys, GI tract, brain, lungs, etc.) are recorded and a maximum tolerated dose (MTD) identified. Harvested tissues are preserved in formalin for histopathology work.

PK study: Serum samples from 6 timepoints (3 mice/timepoint) are analysed using a sandwich ELISA capture assay. The studies will be conducted with IP administration, standard practice for tox studies in mice.

### Preclinical studies in a syngeneic breast cancer model

The ability of AnxA5 conjugates to target and cause shrinkage of a xenografted tumor generated by an isogenic cultured breast cancer cell line (4T1) is determined. BALB/c mice bearing tumors are treated IV with candidate AnxA5 conjugates when the tumor burden reaches its pre-determined half-maximal value. Pharmacological distribution of the conjugates is determined at time of injection and at time of sacrifice. Tumor growth and response to treatment are monitored longitudinally while tumor histology and immune cell composition are determined upon sacrifice. These studies determine that an aggressive breast malignancy can be targeted and diminished in size using conjugates of the invention.

Experimental Design: Mouse breast cancer cells (4T1) are injected into the mammary fat pad of female BALB/c mice and allowed to grow to a half-maximal tumor volume. At that time the mice are randomized into four distinct groups (n=18/group) and treated intravenously with a vehicle control (PBS), unconjugated non-internalizing annexin A5, non-internalizing annexin A5 conjugated to IgG Fc (Anx A5-Fc), or non-internalizing annexin A5 conjugated to TNFα (AnxA5-TNFα) . Mice are monitored on alternate days (weight, tumor dimension and whole animal imaging) until maximal disease burden is achieved in the vehicle control or treatment groups. When maximal disease burden is achieved the animals are sacrificed and processed for further analysis including a final size determination of the tumor, quantitation of the annexin A5 or its conjugates in the tissues and blood, and characterization of the immune cell infiltration into the tumor microenvironment post-treatment.

The 18 mice receiving Anx A5-Fc, the 18 receiving AnxA5-TNFα and the 18 receiving AnxA5 are further sub-divided into 3 groups (n=6) receiving either a low, medium or high dosage determined based on the MTD of the conjugates determined in the Toxicity study.

The study is then repeated using the optimal dosage for each of the constructs: non-internalizing annexin A5, Anx A5-Fc, Anx A5-TNFα or vehicle control (PBS). Each construct is tested again in groups of 18 mice in order to obtain statistically significant results.

### Preclinical studies in a breast cancer induction model

The ability of conjugates according to the invention to target and cause tumor shrinkage in a genetic breast cancer model initiated by the polyoma middle T antigen which is driven by a mouse mammary tumor virus promoter (MMTV-PyMT) is determined. Tumor bearing mice are treated intravenously with candidate AnxA5 conjugates when the tumor burden reaches its pre-determined half-maximal value. Pharmacological distribution of the conjugates is determined at time of injection and at time of sacrifice. Tumor growth and response to treatment are monitored longitudinally while tumor histology and immune cell composition are determined upon sacrifice. Lung metastasis that occurs in this model allows the treatment of both primary and secondary disease to be assessed. Disease progression and disease burden can be diminished using conjugates of the invention.

Experimental Design: Female BALB/c MMTV-PyMT mice are allowed to progress to half-maximal tumor volume. When that tumor burden is reached, mice are selected to participate in four distinct groups and treated intravenously with a vehicle control (PBS), unconjugated non-internalizing annexin A5, non-internalizing annexin A5 conjugated to IgG Fc (Anx A5-Fc), or non-internalizing annexin A5 conjugated to TNFα (AnxA5-TNFα). Mice are monitored on alternate days (weight, tumor dimension) until maximal disease burden is achieved in the vehicle control or treatment groups. When maximal disease burden is achieved the animals are sacrificed and processed for further analysis including a final size determination of the tumor, quantitation of the annexin A5 or its conjugates in the tissues and blood, and characterization of the immune cell infiltration into the tumor microenvironment post-treatment.

18 mice receiving Anx A5-Fc, 18 receiving AnxA5-TNFα and 18 receiving AnxA5 are further sub-divided into 3 groups (n=6) receiving either a low, medium or high dosage determined based on the MTD of the conjugates.

The study is then repeated using the optimal dosage for each of the constructs: non-internalizing annexin A5, Anx A5-Fc, Anx A5-TNFα or vehicle control (PBS). Each construct is tested again in groups of 18 mice in order to obtain statistically significant results.

The invention further includes the subject matter of the claims of WO2016/179430 from which this application is derived, the content of which is reproduced below as numbered paragraphs (paras.) .
1. A conjugate comprising a non-internalising annexin and an immunostimulatory agent, wherein the non-internalising annexin is capable of binding to at least one phospholipid.
2. The conjugate according to 1, wherein the phospholipid is selected from phosphatidylserine (PS), phosphatidyl ethanolamine (PE), phosphatidyl inositol (PI) and phosphoinositidederivatives selected from PIP, PIP2 and PIP3.
3. The conjugate according to 2, wherein the phospholipid is PS.
4. The conjugate according to any one of 1 to 3, wherein the non-internalising annexin is annexin A5.
5. The conjugate according to any one of 1 to 4, wherein one or more amino acids selected from polar amino acids His, Glu, Gln, Asp, Asn, Arg and Lys in the helices IA, ID, IIA, IID, IIIC, IIID and IVE and in the stretches connecting helices IC and ID, IIE and IIIA, IIIC and IIID, IIID and IIIE, and IVA and IVB of wild type annexin are replaced by non-polar amino acids.
6. The conjugate according to any one of the preceding para. , wherein the non-internalising annexin comprises SEQ ID NO:1 for annexin A5 or the corresponding sequences for other annexins wherein the one or more replaced amino acids are located at positions 16-29, 59-74, 88-102, 135-145, 156-169, 202-231, 259-266 and 305-317 of SEQ ID NO:1 for annexin A5, or the corresponding sequences for other annexins.
7. The conjugate according to any one of the preceding para. wherein the non-internalising annexin comprises SEQ ID NO:1 for annexin A5 or the corresponding sequences for other annexins, wherein one or more amino acids selected from Glu, Gln, Asp, Asn, Arg, Lys and His at positions 16-29, 59-74, 88-102, 135-145, 156-169, 202-231, 259-266 and 305-317, or the corresponding sequences for other annexins, are replaced by Gly, Ala, Val, Ile, Leu, Ser, Thr, Met, Pro, Phe, or Tyr.
8. The conjugate according to any one of 5-7, wherein at least two of said polar amino acids are replaced by non-polar amino acids.
9. The conjugate according to any one of the preceding para. wherein the annexin binds to the phospholipid with a dissociation constant of about 10⁻⁶ M or less, preferably 10⁻⁷ M or less, more preferably 10⁻⁸ M or less, even more preferably 10⁻⁹ M or less.
10. The conjugate according to any one of the preceding para. wherein the immunostimulatory agent promotes an inflammatory response.
11. The conjugate according to any one of the preceding para. , wherein the immunostimulatory agent is selected from a nanoparticle, TNFα, IL1α, IL1β, IL2, IL4, IL6, IL8, IL10, IL12, IL15, IL17A, IFNγ, GM-CSF (CSF2), M-CSF (CSF1), G-CSF (CSF 3), an immunoglobulin, and fragments, monomers, multimers, variants, muteins, post-translationally modified versions thereof and mixtures thereof.
12. The conjugate according to 11, wherein the immunostimulatory agent is a fragment crystallizable (Fc) region of an immunoglobulin, monomer or fragment thereof.
13. The conjugate according to 11 or 12, wherein the immunoglobulin is IgG, preferably human IgG1 or IgG3.
14. The conjugate according to 11, wherein the immunostimulatory agent is TNF-α.
15. The conjugate according to any one of the preceding para. , wherein the non-internalising annexin is linked to the immunostimulatory agent via a linker.
16. The conjugate according to 15, wherein the non-internalising annexin is linked to the immunostimulatory agent via the N- or C- terminus, preferably via the N- terminus.
17. The conjugate according to 1, wherein the non-internalising annexin is conjugated to a liposome.
18. The conjugate according to any one of the preceding para. comprising a plurality of immunostimulatory agents and optionally a plurality of linkers.
19. The conjugate according to 18, wherein the immunostimulatory agent is selected from a nanoparticle, TNFα, IL1α, IL1β, IL2, IL4, IL6, IL8, IL10, IL12, IL15, IL17A, IFNγ, GM-CSF (CSF2), M-CSF (CSF1), G-CSF (CSF 3), an immunoglobulin, and fragments, monomers, multimers, variants, muteins, post-translationally modified versions thereof and mixtures thereof.
20. A polypeptide comprising a conjugate according to any one of the preceding para. .
21. A nucleotide sequence encoding a polypeptide according to 20.
22. A conjugate, polypeptide or nucleotide sequence according to any one of 1-21 for use in therapy.
23. A conjugate, polypeptide or nucleotide sequence according to any one of 1-22 for use in a method of treating cancer.
24. A conjugate, polypeptide or nucleotide sequence for the use according to 23, wherein the cancer cells display extracellular PS, PI or PI derivatives selected from PIP, PIP2 and PIP3; or display a different quantity of extracellular PE compared to healthy, normal mammalian cells.
25. A conjugate for the use according to 23 or 24, wherein the conjugate or polypeptide is capable of blocking the immunosuppressive effect of PS on the surface of cancer cells and causes immunostimulation.
26. A conjugate for the use according to any one of 23 to 25, wherein the cancer is a solid tumour.
27. A conjugate for the use according to 26, wherein the tumour is selected from breast, triple negative breast, ovarian , prostate, castrate-resistant prostate, pancreatic, bladder, bone, head and neck, lung, liver, thyroid, esophageal, stomach, intestinal, brain, glioblastoma.
28. A method of treating cancer comprising administering to a patient in need thereof an effective amount of a conjugate comprising a non-internalising annexin and an immunostimulatory agent, wherein the non-internalising annexin is capable of binding to at least one phospholipid.
29. The method according to 28, wherein the phospholipid is selected from phosphatidylserine (PS), phosphatidyl ethanolamine (PE), phosphatidyl inositol (PI) and phosphoinositide derivatives selected from PIP, PIP2 and PIP3.
30. The method according to 28, wherein the phospholipid is PS.
31. The method according to 28, wherein the non-internalising annexin is annexin A5.
32. The method according to 28, wherein one or more amino acids selected from polar amino acids His, Glu, Gln, Asp, Asn, Arg and Lys in the helices IA, ID, IIA, IID, IIIC, IIID and IVE and in the stretches connecting helices IC and ID, IIE and IIIA, IIIC and IIID, IIID and IIIE, and IVA and IVB of wild type annexin are replaced by non-polar amino acids.
33. The method according to 28, wherein the annexin comprises SEQ ID NO:1 for annexin A5 or the corresponding sequences for other annexins and wherein the one or more replaced amino acids are located at positions 16-29, 59-74, 88-102, 135-145, 156-169, 202-231, 259-266 and 305-317 of SEQ ID NO:1 for annexin A5, or the corresponding sequences for other annexins.
34. The method according to 28, wherein the non-internalising annexin comprises SEQ ID NO:1 for annexin A5 or the corresponding sequences for other annexins, wherein one or more amino acids selected from Glu, Gln, Asp, Asn, Arg, Lys and His at positions 16-29, 59-74, 88-102, 135-145, 156-169, 202-231, 259-266 and 305-317, or the corresponding sequences for other annexins, are replaced by Gly, Ala, Val, Ile, Leu, Ser, Thr, Met, Pro, Phe, or Tyr.
35. The method according to any one of 32-34, wherein at least two of said polar amino acids are replaced by non-polar amino acids.
36. The method according to 28, wherein the annexin binds to the phospholipid with a dissociation constant of about 10⁻⁶ M or less, preferably 10⁻⁷ M or less, more preferably 10⁻⁸ M or less, even more preferably 10⁻⁹ M or less.
37. The method according to 28, wherein the immunostimulatory agent promotes an inflammatory response.
38. The method according tc 28, wherein the immunostimulatory agent is selected from TNFα, IL1α, IL1β, IL2, IL4, IL6, IL8, IL10, IL12, IL15, IL17A, IFNγ, GM-CSF (CSF2), M-CSF (CSF1), G-CSF (CSF 3), an immunoglobulin, and fragments, monomers, multimers, variants, muteins, post-translationally modified versions thereof and mixtures thereof.
39. The method according to 28, wherein the immunostimulatory agent is a fragment crystallizable (Fc) region of an immunoglobulin, monomer or fragment thereof.
40. The method according to 39, wherein the immunoglobulin is IgG, preferably human IgG1 or IgG3.
41. The method according to 28, wherein the immunostimulatory agent is TNF-α.
42. The method according to 28, wherein the non-internalising annexin is linked to the immunostimulatory agent via a linker.
43. The method according to 42, wherein the non-internalising annexin is linked to the immunostimulatory agent via the N- or C- terminus, preferably via the N- terminus.
44. The method according to 28, wherein the conjugate comprises a plurality of immunostimulatory agents and optionally a plurality of linkers.
45. The method according to 44, wherein the immunostimulatory agent is selected from a nanoparticle, TNFα, IL1α, IL1β, IL2, IL4, IL6, IL8, IL10, IL12, IL15, IL17A, IFNγ, GM-CSF (CSF2), M-CSF (CSF1), G-CSF (CSF 3), an immunoglobulin, and fragments, monomers, multimers, variants, muteins, post-translationally modified versions thereof and mixtures thereof.
46. The method according to 28, wherein a cancer cells display extracellular PS, PI or PI derivatives selected from PIP, PIP2 and PIP3; or displays a different quantity of extracellular PE compared to a healthy, normal mammalian cell.
47. The method according tc 28, wherein the conjugate is capable of blocking the immunosuppressive effect of PS on the surface of cancer cells and causes immunostimulation.
48. The method according to 28, wherein the cancer is a solid tumour.
49. The method according to 48, wherein the tumour is selected from breast, triple negative breast, ovarian , prostate, castrate-resistant prostate, pancreatic, bladder, bone, head and neck, lung, liver, thyroid, esophageal, stomach, intestinal, brain, glioblastoma.

## Claims

1. (a) A conjugate comprising:
(i) a non-internalising variant of an annexin, wherein the non-internalising annexin variant comprises one or more polar-to-nonpolar amino acid substitutions and is capable of binding to at least one phospholipid; and
(ii) an immunostimulatory agent capable of stimulating an immune response against a solid tumour in a subject;
(b) a polypeptide comprising a conjugate according to (a); or
(c) a nucleotide sequence encoding a polypeptide according to (b) for use in a method of treating a cancer.

2. The conjugate, polypeptide, or nucleotide sequence for use according to claim 1, wherein the phospholipid is selected from phosphatidylserine (PS), phosphatidyl ethanolamine (PE), phosphatidyl inositol (PI) and phosphoinositide derivatives selected from PIP, PIP2 and PIP3.

3. The conjugate, polypeptide, or nucleotide sequence for use according to claim 1, wherein the phospholipid is PS.

4. The conjugate, polypeptide, or nucleotide sequence for use according to claim 1, wherein the non-internalising annexin variant is a non-internalising variant of annexin A5.

5. The conjugate, polypeptide, or nucleotide sequence for use according to claims 1, wherein the one or more polar-to-nonpolar amino acid substitutions is selected from polar amino acids His, Glu, Gln, Asp, Asn, Arg and Lys in the helices IA, ID, IIA, IID, IIIC, IIID and IVE and in the stretches connecting helices IC and ID, IIE and IIIA, IIIC and IIID, IIID and IIIE, and IVA and IVB of the annexin sequence.

6. The conjugate, polypeptide, or nucleotide sequence for use according to claim 1, wherein the non-internalising annexin variant comprises one or more polar-to-nonpolar amino acid substitutions at positions corresponding to amino acid positions 16-29, 59-74, 88-102, 135-145, 156-169, 202-231, 259-266 and 305-317 of SEQ ID NO: 1.

7. The conjugate, polypeptide, or nucleotide sequence for use according to claim 1, wherein the one or more polar-to-nonpolar amino acid substitutions is selected from Glu, Gln, Asp, Asn, Arg, Lys and His at positions corresponding to amino acid positions 16-29, 59-74, 88-102, 135-145, 156-169, 202-231, 259-266 and 305-317 of SEQ ID NO: 1 that have been replaced by Gly, Ala, Val, Ile, Leu, Ser, Thr, Met, Pro, Phe, or Tyr.

8. The conjugate, polypeptide, or nucleotide sequence for use according to any one of claims 5-7, wherein the non-internalising annexin variant comprises at least two polar-to-nonpolar amino acid substitutions.

9. The conjugate, polypeptide, or nucleotide sequence for use according to claim 1, wherein (a) the non-internalising annexin variant binds to the phospholipid with a dissociation constant of about 10⁻⁶ M or less, preferably 10⁻⁷ M or less, more preferably 10⁻⁸ M or less, even more preferably 10⁻⁹ M or less; or (b) the immunostimulatory agent promotes an inflammatory response.

10. The conjugate, polypeptide, or nucleotide sequence for use according to claim 1, wherein the immunostimulatory agent is selected from the group consisting of a nanoparticle, TNF-α, IL1α, IL1β, IL2, IL4, IL6, IL8, IL1β, IL12, IL15, IL17A, IFNy, GM-CSF (CSF2), M-CSF (CSF1), G-CSF (CSF 3), an immunoglobulin; and fragments, monomers, multimers, variants, muteins, and post-translationally modified versions of any thereof,
optionally wherein (a) the immunostimulatory agent is a fragment crystallizable (Fc) region of an immunoglobulin, monomer or fragment thereof, optionally wherein the immunoglobulin is IgG, preferably human IgG1 or IgG3; or
(b) wherein the immunostimulatory agent is TNF-α.

11. The conjugate, polypeptide, or nucleotide sequence for use according to claim 1, wherein the non-internalising annexin variant is linked to the immunostimulatory agent via a linker, optionally wherein the non-internalising annexin variant is linked to the immunostimulatory agent via the N- or C- terminus, preferably via the N- terminus.

12. The conjugate, polypeptide, or nucleotide sequence for use according to claim 1, wherein the conjugate comprises a plurality of immunostimulatory agents and optionally a plurality of linkers,
optionally wherein the immunostimulatory agent is selected from a nanoparticle, TNF-α, IL1α, IL1β, IL2, IL4, IL6, IL8, IL10, IL12, IL15, IL17A, IFNγ, GM-CSF (CSF2), M-CSF (CSF1), G-CSF (CSF 3), an immunoglobulin, and fragments, monomers, multimers, variants, muteins, post-translationally modified versions thereof and mixtures thereof.

13. The conjugate, polypeptide, or nucleotide sequence for use according to claim 1, wherein the cancer cells display extracellular PS, PI or PI derivatives selected from PIP, PIP2 and PIP3; or display a different quantity of extracellular PE compared to healthy, normal mammalian cells.

14. The conjugate, polypeptide, or nucleotide sequence for use according to any one of claims 1 to 13, wherein the conjugate or polypeptide is capable of blocking the immunosuppressive effect of PS on the surface of cancer cells and causes immunostimulation.

15. The conjugate, polypeptide, or nucleotide sequence for use according to any one of claims 1 to 14, wherein the cancer is a solid tumour, optionally wherein the tumour is selected from breast, triple negative breast, ovarian , prostate, castrate-resistant prostate, pancreatic, bladder, bone, head and neck, lung, liver, thyroid, esophageal, stomach, intestinal, brain, glioblastoma.
